# EUROPEAN PATENT APPLICATION

(11) **EP 4 108 197 A1**
(43) Date of publication of application: **28.12.2022**
(21) Application number: 21305873.8
(22) Date of filing: 24.06.2021
(51) Int. Cl.: A61B 18/14, A61N 7/02, A61N 7/00, A61B 90/00

(54) **SYSTEMS FOR TREATING TISSUE**

(71) Applicant: Gradient Denervation Technologies, 75009 Paris (FR)
(72) Inventor: AMAOUA, David, 78000 VERSAILLES (FR); MAXWELL, Adam, Douglas, WASHINGTON, 98036 (US); MISCHO, Chiara, Dundalk, A91P9VK (IE); CANNON, William, Galway, H91YYC5 (IE)
(74) Representative: Brevalex

(57) **Abstract**

A catheter for ablation of tissue around a vessel. The catheter includes an elongate shaft comprising a distal portion. The distal portion includes a transducer and an anchor. The anchor is changeable between a collapsed state and a deployed state. The anchor in the deployed state is configured to center the transducer in a vessel, to maintain a longitudinal position of the transducer in the vessel, and to preserve blood flow through the vessel.

## Description

### BACKGROUND

### Field

The present application is directed generally to medical devices, systems and methods for treating tissue. More specifically, the application is directed to devices, systems and methods for treating nerves, nerve fibers, or neurons to treat pulmonary hypertension and/or other pulmonary vascular disorders.

### Description of the Related Art

Pulmonary hypertension is a disease phenomenon of multifactorial etiology with high morbidity and mortality. The disease causes increased work for the right side of the heart and eventually hypertrophy and dysfunction of not only the right side of the heart, but often the left side as well. The prognosis of pulmonary hypertension historically has been poor, with median survival historically being less than 3 years Currently, with the advent of new pharmacologic therapies, survival has improved to 50 to 60% at 5 years. However, many patients continue to progress to worsening stages of pulmonary hypertension, and despite improvements in therapy, prognosis for the condition remains grave.

### SUMMARY

It is desirable to have new treatments for pulmonary hypertension. Preferably, such treatments would be minimally invasive, would reduce or negate the need for pharmaceutical remedies, and/or would be permanent or at least long-lasting.

Interrupting the nerves (e.g., sympathetic nerves) around and/or innervating the left, right, and/or main pulmonary arteries, according to several embodiments described herein, can reduce pulmonary hypertension. In some embodiments, neuromodulation is accomplished (e.g., via ablation, denervation, which may or may not be reversible, stimulation, etc.). For example, methods and systems have been developed to navigate a catheter from a remote insertion point, through the heart, and into the pulmonary branch arteries and trunk. The catheter includes an anchor that, when deployed, will anchor a transducer, for example parallel to vessel walls and/or centered, at an ablation site. Once the nerves located at the ablation site have been ablated, the anchor is collapsed, and the transducer is pushed or pulled to the next ablation site within the vessel. This deploy, ablate, collapse, and move method will be repeated until both pulmonary artery branches and the pulmonary trunk have been ablated.

In some embodiments, a catheter for ablation of tissue around a vessel comprises, or alternatively consists essentially of, an elongate shaft comprising a distal portion. The distal portion comprises a transducer and an anchor. The anchor is changeable between a collapsed state and a deployed state. The anchor in the deployed state is configured to center the transducer in a vessel and to maintain a longitudinal position of the transducer in the vessel. The anchor is configured to preserve blood flow through the vessel.

The anchor may be self-expanding. The anchor may be deployable and collapsible via a push-pull mechanism. The anchor may comprise a plurality of struts surrounding the transducer. The transducer may comprise a plurality of arcuate sections. The elongate shaft may comprise an intermediate portion between the proximal portion and the distal portion, and a suspension between the intermediate portion and the distal portion. The suspension may be more flexible than the intermediate portion and the distal portion. The suspension may be configured to reduce shaft stiffness. The suspension may comprise at least one of a coil or a flexible material. The distal portion may have a diameter between 3 mm and 10 mm. The distal portion may comprise a lens. The lens may comprise a Fresnel lens. The lens may be a Fresnel lens.

In some embodiments, a catheter for ablation of tissue around a vessel comprises, or alternatively consists essentially of, an elongate shaft comprising a distal portion. The distal portion comprises a transducer, a Fresnel lens disposed on the transducer, and an anchor. The anchor is changeable between a collapsed state and a deployed state. The anchor in the deployed state is configured to center the transducer in a vessel and to maintain a longitudinal position of the transducer in the vessel.

The anchor may be self-expanding. The anchor may be deployable and collapsible via a push-pull mechanism. The anchor may comprise a plurality of struts surrounding the transducer. The transducer may comprise a plurality of arcuate sections. The elongate shaft may comprise an intermediate portion between the proximal portion and the distal portion, and a suspension between the intermediate portion and the distal portion. The suspension may be more flexible than the intermediate portion and the distal portion. The suspension may be configured to reduce shaft stiffness. The suspension may comprise at least one of a coil or a flexible material. The distal portion may have a diameter between 3 mm and 10 mm. The anchor may be configured to preserve blood flow through the vessel.

In some embodiments, a denervation system may comprise the catheter and an ultrasound system comprising a generator configured to generate an output signal having a frequency between 1.5 MHz and 11 MHz. The ultrasound system may be configured to adjust the output signal based on data regarding efficiency of the distal portion.

In some embodiments, a catheter for ablation of tissue around a vessel comprises, or alternatively consists essentially of, an elongate shaft, an outer sheath, and a handle coupled to a proximal portion of the elongate shaft. The elongate shaft comprises a distal portion having a delivery state and a deployment state. The distal portion comprises an arcuate ultrasound transducer, a self-expanding anchor, and marker bands. The anchor in the deployed state is configured to center the transducer in a vessel and to maintain a longitudinal position of the transducer in the vessel. The outer sheath surrounds the anchor in the delivery state. The handle comprises an actuator configured to retract the outer sheath. Retracting the outer sheath allows the anchor to self-expand from the delivery state to the deployed state confirmable by a change in position of the marker bands.

The elongate shaft may have a diameter between 4.5 mm and 8.5 mm. The anchor may be deployable and collapsible via a push-pull mechanism. The anchor may comprise a plurality of struts surrounding the transducer. The distal portion may comprise a lens disposed on the transducer. The lens may comprise a Fresnel lens. The lens may be a Fresnel lens. The elongate shaft may comprise an intermediate portion between the proximal portion and the distal portion, and a suspension between the intermediate portion and the distal portion. The suspension may be more flexible than the intermediate portion and the distal portion. The suspension may be configured to reduce shaft stiffness. The anchor may be configured to preserve blood flow through the vessel.

In some embodiments, a catheter for ablation of tissue around a vessel comprises, or alternatively consists essentially of, an elongate shaft, an outer sheath, and a handle coupled to a proximal portion of the elongate shaft. The elongate shaft comprises a distal portion having a delivery state and a deployment state. The distal portion comprises an arcuate ultrasound transducer, a Fresnel lens disposed on the transducer, a self-expanding anchor, and marker bands. The anchor in the deployed state is configured to center the transducer in a vessel and to maintain a longitudinal position of the transducer in the vessel. The outer sheath surrounds the anchor in the delivery state. The handle comprises an actuator configured to retract the outer sheath. Retracting the outer sheath allows the anchor to self-expand from the delivery state to the deployed state confirmable by a change in position of the marker bands.

The elongate shaft may have a diameter between 4.5 mm and 8.5 mm. The anchor may comprise a plurality of struts surrounding the transducer. Each of the plurality of struts may have a thickness between 50 µm and 100 µm. The anchor may comprise a first braid section and a second braid section. The transducer may be located between the first braid section and the second braid section. The anchor may comprise a first plurality of struts and a second plurality of struts. The transducer may be located between the first and the second section of struts. The anchor may comprise a first plurality of petals located adjacent the transducer. The anchor may further comprise a second plurality of petals. The transducer may be located between the first plurality of petals and the second plurality of petals. The anchor may comprise a first loop wire located adjacent the transducer. The anchor may further comprise a second loop wire. The transducer may be located between the first loop wire and the second loop wire. The transducer may comprise a plurality of longitudinal sections spaced in the delivery state. The transducer may comprise a plurality of arcuate sections. The transducer may comprise at least six sections. The transducer may be configured to detect efficacy of the ablation. The distal portion of the elongate shaft may comprise a sensor. The sensor may comprise at least one of a temperature sensor or a pressure sensor. The transducer may comprise the sensor. The elongate shaft may comprise an intermediate portion between the proximal portion and the distal portion, and a suspension between the intermediate portion and the distal portion. The suspension may be more flexible than the intermediate portion and the distal portion. The suspension may be configured to reduce shaft stiffness. The suspension may comprise a coil. The suspension may comprise flexible material. The distal portion may have a diameter between 3 mm and 10 mm. The anchor may be configured to preserve blood flow through the vessel.

A denervation system may comprise the catheter and an ultrasound system. The ultrasound system may comprise a generator configured to generate an output signal having a frequency between 1.5 MHz and 11 MHz, a power monitor configured to monitor the emitted power, and a control computer connected to the catheter. The control computer may comprise a user interface and a planning tool configured to assist the operator. The control computer may be connected to the catheter by a directional coupler. The ultrasound system may be configured to adjust the output signal based on data regarding efficiency of the distal portion.

In some embodiments, a catheter for ablation of tissue around a vessel, the catheter comprises, or alternatively consists essentially of, an elongate shaft and a handle coupled to a proximal portion of the elongate shaft. The elongate shaft comprises a distal portion. The distal portion comprises a transducer, and an anchor. The anchor in the deployed state is configured to center the transducer in a vessel and to maintain a longitudinal position of the transducer in the vessel. The handle comprises an actuator configured to deploy the anchor.

The distal portion may comprise a lens. The lens may be disposed on the transducer. The lens may comprise a Fresnel lens. The lens may be a Fresnel lens. The anchor may be deployed and/or collapsed via a push-pull mechanism. The anchor may comprise a plurality of struts surrounding the transducer. Each of the plurality of struts may have a thickness between 50 µm and 100 µm. The anchor may comprise a first plurality of struts and a second plurality of struts. The transducer may be located between the first and the second section of struts. The anchor may comprise a first braid section and a second braid section. The transducer may be located between the first braid section and the second braid section. The anchor may comprise a first plurality of petals may be located adjacent the transducer. The anchor may further comprise a second plurality of petals. The transducer may be located between the first plurality of petals and the second plurality of petals. The anchor may comprise a first loop wire. The first wire loop may be located adjacent the transducer. The anchor may further comprise a second loop wire. The transducer may be located between the first loop wire and the second loop wire. The transducer may comprise a plurality of longitudinal sections spaced in the delivery state. The transducer may comprise a plurality of arcuate sections. The transducer may comprise at least six sections. The distal portion may comprise a sensor. The sensor may comprise at least one of a temperature sensor or a pressure sensor. The transducer may comprise the sensor. The elongate shaft may comprise an intermediate portion between the proximal portion and the distal portion, and a suspension between the intermediate portion and the distal portion. The suspension may be more flexible than the intermediate portion and the distal portion. The suspension may be configured to reduce shaft stiffness. The suspension may comprise a coil. The suspension may comprise a flexible material. The distal portion may have a diameter between 3 mm and 12 mm. The anchor may be configured to preserve blood flow through the vessel.

In some embodiments, a catheter for ablation of tissue around a vessel comprises, or alternatively consists essentially of, an elongate shaft comprising a distal portion comprising a transducer, a Fresnel lens disposed on the transducer, and an anchor. The anchor is changeable between a collapsed state and a deployed state. The anchor in the deployed state is configured to center the transducer in a vessel and to maintain a longitudinal position of the transducer in the vessel.

The anchor may be self-expanding. The anchor may be deployable and collapsible via a push-pull mechanism. The anchor may comprise a plurality of struts surrounding the transducer. Each of the plurality of struts may have a thickness between 50 µm and 100 µm. The anchor may comprise a first plurality of struts and a second plurality of struts. The transducer may be located between the first and the second section of struts. The anchor may comprise a first braid section and a second braid section. The transducer may be located between the first braid section and the second braid section. The anchor may comprise a first plurality of petals. The first plurality of petals may be located adjacent the transducer. The anchor may further comprise a second plurality of petals. The transducer may be located between the first plurality of petals and the second plurality of petals. The anchor may comprise a first loop wire. The first loop wire may be located adjacent the transducer. The anchor may further comprise a second loop wire. The transducer may be located between the first loop wire and the second loop wire. The transducer may comprise a plurality of longitudinal sections spaced in the delivery state. The transducer may comprise a plurality of arcuate sections. The transducer may comprise at least six sections. The distal portion of the elongate shaft further may comprise a sensor. The sensor may comprise at least one of a temperature sensor or a pressure sensor. The transducer may comprise the sensor. The elongate shaft may comprise an intermediate portion between the proximal portion and the distal portion, and a suspension between the intermediate portion and the distal portion. The suspension may be more flexible than the intermediate portion and the distal portion. The suspension may be configured to reduce shaft stiffness. The suspension may comprise a coil. The suspension may comprise a flexible material. The distal portion may have a diameter between 3 mm and 12 mm. The anchor may be configured to preserve blood flow through the vessel.

In some embodiments, a method for treating pulmonary hypertension comprises providing the catheter, positioning the catheter into a first pulmonary branch artery, deploying the anchor in the first pulmonary branch artery, ablating a first section of the first pulmonary branch artery, collapsing the anchor, retracting the catheter a distance, and repeating the deploying, ablating, collapsing and retracting steps until an amount of the first pulmonary branch artery has been covered by ablation.

The method may comprise positioning the treatment catheter in the trunk of the pulmonary artery, and repeating the deploying, ablating, collapsing and retracting steps until an amount of the trunk of the pulmonary artery may have been covered by ablation. The method may comprise positioning the treatment catheter in a second pulmonary branch artery and repeating the deploying, ablating, collapsing and retracting steps until an amount of the second pulmonary branch artery may have been covered by ablation.

The method may comprise removing the treatment catheter. The amount of the first pulmonary branch artery may be between half of the first pulmonary branch artery and an entirety of the first pulmonary branch artery. The amount of the second pulmonary branch artery may be between half of the second pulmonary branch artery and an entirety of the second pulmonary branch artery. The amount of the trunk of the pulmonary artery may be between half of the trunk of the pulmonary artery and an entirety of the trunk of the pulmonary artery. A number of repetitions of the deploying, ablating, collapsing and retracting steps in the first pulmonary branch artery may be between two and ten. A number of repetitions of the deploying, ablating, collapsing and retracting steps in the second pulmonary branch artery may be between two and ten. A number of repetitions of the deploying, ablating, collapsing and retracting steps in the trunk of the pulmonary artery may be between two and ten. The distance may comprise between 0.25 cm and 2 cm. The elongate shaft may comprise markings at predetermined intervals. The elongate shaft may comprise electrodes at predetermined intervals. The anchor may be self-expanding. Deploying the anchor may comprise retracting an outer sheath. Deploying the anchor may comprise pulling a wire or a tube. Collapsing the anchor may comprise advancing an outer sheath. Collapsing the anchor may comprise pushing a wire or a tube. The method may comprise allowing blood to flow through the vessel when the anchor is in the deployed state.

In some embodiments, a denervation system comprises, or alternatively consists essentially of, a catheter comprising an elongate shaft having a distal portion comprising a transducer and an ultrasound system. The ultrasound system comprises a generator configured to generate an output signal having a frequency between 1.5 MHz and 11 MHz, a power monitor configured to monitor the emitted power through the use of a directional coupler, and a control computer connected to the catheter. The control computer comprises a user interface and a planning tool configured to assist the operator.

The distal portion of the elongate shaft of the catheter may comprise a lens. The lens may comprise a Fresnel lens. The lens may be a Fresnel lens. The distal portion of the elongate shaft of the catheter may comprise an anchor having a deployed state and a delivery state. The anchor in the deployed state may be configured to center the transducer in a vessel and/or to maintain a longitudinal position of the transducer in the vessel. The anchor may be deployed and collapsed via a push-pull mechanism. The anchor may comprise a plurality of struts surrounding the transducer. Each of the plurality of struts may have a thickness between 50 µm and 100 µm. The distal portion of the elongate shaft may comprise a sensor. The sensor may comprise at least one of a temperature sensor or a pressure sensor. The transducer may comprise the sensor. The anchor may be configured to preserve blood flow through the vessel.

In some embodiments, a method for treating pulmonary hypertension comprises positioning a first catheter in a vein. The first catheter comprises a first elongate shaft and a balloon along a distal section of the first elongate shaft. The method further comprises, after positioning the first catheter in the vein, inflating the balloon, allowing the balloon to be carried by blood to a first pulmonary branch artery, inserting a guidewire to the first pulmonary branch artery, retracting the first catheter from the vein, and advancing a treatment catheter over the guidewire to the first pulmonary branch artery. The treatment catheter comprises a second elongate shaft having a distal portion. The distal portion comprises an arcuate ultrasound transducer, a Fresnel lens disposed on the transducer, and an anchor. The method further comprises deploying the anchor in the first pulmonary branch artery. The anchor in the deployed state is configured to center the transducer in the first pulmonary branch artery and to maintain a longitudinal position of the transducer in the first pulmonary branch artery. The method further comprises ablating a first section of the first pulmonary branch artery, collapsing the anchor, retracting the catheter a distance, and repeating the deploying, ablating, collapsing and retracting steps until an amount of the first pulmonary branch artery has been covered by ablation. The method further comprises positioning the treatment catheter in the trunk of the pulmonary artery and repeating the deploying, ablating, collapsing and retracting steps until an amount of the trunk of the pulmonary artery has been covered by ablation. The method further comprises positioning the treatment catheter in a second pulmonary artery and repeating the deploying, ablating, collapsing and retracting steps until an amount of the second pulmonary artery has been covered by ablation.

The method may comprise removing the treatment catheter. The amount of the first pulmonary branch artery may be between half of the first pulmonary branch artery and an entirety of the first pulmonary branch artery. The amount of the second pulmonary branch artery may be between half of the second pulmonary branch artery and an entirety of the second pulmonary branch artery. The amount of the trunk of the pulmonary artery may be between half of the trunk of the pulmonary artery and an entirety of the trunk of the pulmonary artery. A number of repetitions of the deploying, ablating, collapsing and retracting steps in the first pulmonary branch artery may be between two and ten. A number of repetitions of the deploying, ablating, collapsing and retracting steps in the second pulmonary branch artery may be between two and ten. A number of repetitions of the deploying, ablating, collapsing and retracting steps in the trunk of the pulmonary artery may be between two and ten. The distance may comprise between 0.25 cm and 2 cm. The second elongate shaft may comprise markings at predetermined intervals. The second elongate shaft may comprise electrodes at predetermined intervals. The anchor may be self-expanding. Deploying the anchor may comprise retracting an outer sheath. Deploying the anchor may comprise pulling a wire or a tube. Collapsing the anchor may comprise advancing an outer sheath. Collapsing the anchor may comprise pushing a wire or a tube. The method may comprise allowing blood to flow through the vessel when the anchor is in the deployed state.

In some embodiments, a method for treating pulmonary hypertension comprises advancing a treatment catheter to a vessel. The treatment catheter comprises an elongate shaft having a distal portion. The distal portion comprises an arcuate ultrasound transducer, a lens disposed on the transducer, and an anchor. The method further comprises deploying the anchor in the vessel. The anchor in the deployed state is configured to center the transducer in the vessel and to maintain a longitudinal position of the transducer in the vessel. The method further ablating a first section of the vessel, collapsing the anchor, and moving the catheter a distance. The method may comprise repeating the deploying, ablating, collapsing, and moving steps until an amount of the vessel has been covered by ablation. The method may comprise removing the treatment catheter.

The vessel may comprise a pulmonary artery. The vessel may comprise a renal artery. The amount of the vessel may be between half of the artery and an entirety of the artery. A number of repetitions of the deploying, ablating, collapsing, and moving steps in the vessel may be between two and ten. The distance may comprise between 0.25 cm and 2 cm. The second elongate shaft may comprise markings at predetermined intervals. The second elongate shaft may comprise electrodes at predetermined intervals. The anchor may be self-expanding. Deploying the anchor may comprise retracting an outer sheath. Deploying the anchor may comprise pulling a wire or a tube. Collapsing the anchor may comprise advancing an outer sheath. Collapsing the anchor may comprise pushing a wire or a tube. The method may comprise advancing the treatment catheter to a second vessel different than the first vessel (e.g., right pulmonary artery is different than left pulmonary artery, left renal artery is different than right renal artery, etc.), deploying the anchor in the second vessel, ablating a first section of the second vessel, collapsing the anchor, and moving the catheter a distance. The method may comprise repeating the deploying, ablating, collapsing, and moving steps until an amount of the second vessel has been covered by ablation. The method may comprise allowing blood to flow through the vessel when the anchor is in the deployed state.

### BRIEF DESCRIPTION OF THE DRAWINGS

Fig. 1 is a schematic illustration of a human heart with the pulmonary trunk and right and left pulmonary arteries branching from the pulmonary trunk, along with an example catheter located in the left pulmonary artery.
Fig. 2A illustrates an example transducer.
Fig. 2B illustrates another example transducer.
Fig. 2C illustrates another example transducer.
Fig. 2D illustrates an example transducer connection implementation.
Fig. 2E illustrates another example transducer.
Figs. 3A-3D illustrate example outer surface shapes of example transducers.
Fig. 4A illustrates an example lens.
Fig. 4B schematically illustrates example energy rays emanating from the lens of Fig. 4A to longitudinally focus and concentrate energy.
Fig. 4C illustrates an example portion of an energy application shape.
Fig. 4D illustrates an example transducer assembly.
Fig. 5A illustrates an example anchor in a collapsed or delivery state.
Fig. 5B illustrates the example anchor of Fig. 5A in a deployed state.
Fig. 5C illustrates another example anchor in a collapsed or delivery state.
Fig. 5D illustrates the example anchor of Fig. 5C in a deployed state.
Figs. 6A and 6B illustrate an example transducer assembly.
Figs. 6C-6E illustrate an example method of rotating an anchor between ablations.
Fig. 7A schematically illustrates an example embodiment of a catheter comprising a handle and an elongate shaft.
Fig. 7B schematically illustrates another example embodiment of a catheter comprising a handle and an elongate shaft.
Fig. 8A illustrates another example anchor in a collapsed or delivery state.
Fig. 8B illustrates the example anchor of Fig. 8A in a deployed state.
Fig. 9A illustrates another example anchor in a collapsed or delivery state.
Fig. 9B illustrates the example anchor of Fig. 9A in a deployed state.
Fig. 10A illustrates another example anchor in a collapsed or delivery state.
Fig. 10B illustrates the example anchor of Fig. 10A in a deployed state.
Fig. 10C is a top view of an example petal configuration for the anchor of Fig. 10A.
Fig. 10D is a side view of the example petal configuration of Fig. 10C.
Fig. 11A illustrates another example anchor in a collapsed or delivery state.
Fig. 11B illustrates the example anchor of Fig. 11A in a deployed state.
Fig. 11C illustrates another example anchor in a collapsed or delivery state.
Fig. 11D illustrates the example anchor of Fig. 11C in a deployed state.
Fig. 12A illustrates an example anchor in a collapsed or delivery state.
Fig. 12B illustrates the example anchor of Fig. 12A in a deployed state.
Fig. 12C illustrates a top view of an example loop wire.
Fig. 12D is a picture of an example loop wire.
Fig. 13A illustrates an example catheter in a vessel that is not properly anchored.
Fig. 13B illustrates an example embodiment of a catheter in which the stiffness of a shaft can be effectively negated proximate a distal portion.
Fig. 14A illustrates an example retraction feature.
Fig. 14B illustrates another example retraction feature.
Fig. 15 is a schematic diagram of an example ablation instrument.
Fig. 16A illustrates an example catheter comprising a sensor.
Fig. 16B is a graph depicting example temperature measurement and pulse emission during ablation.
Fig. 16C illustrates an example catheter system including a second catheter comprising a sensor.
Fig. 16D illustrates a sensor coupled to the interior of a lens.
Fig. 16E illustrates a plurality of sensors located on an anchor.
Figs. 17A-17B illustrate an example method of inserting and navigating a catheter to a vessel.
Figs. 17C-17D illustrate an example method of treating tissue around the right pulmonary artery.
Figs. 17E-17F illustrate an example method of treating tissue around the left pulmonary artery.
Figs. 17G-17I illustrate an example method of treating tissue around the pulmonary trunk.

### DETAILED DESCRIPTION

The interplay of the vasoconstrictive/vasodilator axis of the pulmonary circulation is one of the key determinants of pulmonary hypertension disease progression and severity. The sympathetic nervous system mediates pulmonary vasoconstriction. This may be specifically accomplished by the thoracic sympathetic chain and branches thereof. The sympathetic nervous system can be important in the mediation of the hypoxia mediated vasoconstrictive response of the pulmonary arterial vasculature. Modulating or reducing the sympathetic nervous system activity within the pulmonary vasculature is a unique approach for the treatment of pulmonary hypertension. Reducing or modulating or negating sympathetic tone to the pulmonary arteries reduces sympathetic mediated vasoconstriction, thereby allowing for increased pulmonary vascular diameter and pulmonary vascular dilatation. The end effect of reducing sympathetic tone is a reduction in pulmonary pressure and pulmonary hypertension, a possible goal of therapy.

Although this Detailed Description focuses on treatment of sympathetic nerves, nerve fibers and/or neurons, in any given embodiment, a method, device or system described herein may also or alternatively treat parasympathetic nerves, nerve fibers, and/or neurons. Therefore, descriptions herein of treating sympathetic nervous tissue should not be interpreted as limiting.

### Pulmonary Neurovascular Anatomy

The sympathetic innervation of the lung and the heart arises from the thoracolumbar spinal column, ultimately reaching the heart and lung and innervating its vasculature. The sympathetic nervous system is part of the autonomic nervous system, comprising nerve fibers that leave the spinal cord in the thoracic and lumbar regions and supply viscera and blood vessels by way of a chain of sympathetic ganglia running on each side of the spinal column which communicate with the central nervous system via a branch to a corresponding spinal nerve. The sympathetic nerves arising from primarily the thoracic spine (e.g., levels T1-T10 with some potential contribution from the cervical spine) innervate the heart and the lungs after branching out from the thoracic sympathetic chain. The sympathetic nerves converge upon the thoracic sympathetic chain and ganglion, after which arise the post ganglionic sympathetic nerves which then innervate the heart and the lungs. These nerves often converge upon various plexi or plexuses which are areas of convergence often of both sympathetic and parasympathetic nerve fibers. These plexuses then further give rise to nerve branches or continuations, which then branch and ramify onto structures within the heart and lungs or in association with the outer walls of the pulmonary arteries or arterioles for instance. Some of the key plexuses and their anatomic relationship to the heart, lung, and pulmonary vasculature are described herein.

The great plexuses of the sympathetic are aggregations of nerves and ganglia, situated in the thoracic, abdominal, and pelvic cavities, and named the cardiac, celiac, and hypogastric plexuses. They include not only sympathetic fibers derived from the ganglia, but also fibers from the medulla spinalis, which are conveyed through the white rami communicantes. From the plexuses, branches are given to the thoracic, abdominal, and pelvic viscera.

The cardiac plexus is situated at the base of the heart, and is divided into a superficial part, which lies in the concavity of the aortic arch, and a deep part, which is between the aortic arch and the trachea. The superficial and deep parts are closely connected.

The superficial part of the cardiac plexus lies beneath the arch of the aorta, in front of the right pulmonary artery. The superficial part of the cardiac plexus is formed by the superior cardiac branch of the left sympathetic and the lower superior cervical cardiac branch of the left vagus. A small ganglion, the cardiac ganglion of Wrisberg, is occasionally found connected with these nerves at their point of junction. This ganglion, when present, is situated immediately beneath the arch of the aorta, on the right side of the ligamentum arteriosum. The superficial part of the cardiac plexus gives branches (a) to the deep part of the plexus; (b) to the anterior coronary plexus; and (c) to the left anterior pulmonary plexus.

The deep part of the cardiac plexus is situated in front of the bifurcation of the trachea, above the point of division of the pulmonary artery, and behind the aortic arch. The deep part of the cardiac plexus is formed by the cardiac nerves derived from the cervical ganglia of the sympathetic and the cardiac branches of the vagus and recurrent nerves. The only cardiac nerves which do not enter into the formation of the deep part of the cardiac plexus are the superior cardiac nerve of the left sympathetic and the lower of the two superior cervical cardiac branches from the left vagus, which pass to the superficial part of the plexus.

The branches from the right half of the deep part of the cardiac plexus pass, some in front of and others behind, the right pulmonary artery; the branches in front of the pulmonary artery, which are more numerous than the branches behind, transmit a few filaments to the anterior pulmonary plexus, and then continue onward to form part of the anterior coronary plexus; those behind the pulmonary artery distribute a few filaments to the right atrium, and then continue onward to form part of the posterior coronary plexus.

The left half of the deep part of the plexus is connected with the superficial part of the cardiac plexus, and gives filaments to the left atrium, and to the anterior pulmonary plexus, and then continues to form the greater part of the posterior coronary plexus.

The Posterior Coronary Plexus (plexus coronarius posterior; left coronary plexus) is larger than the Anterior Coronary Plexus, and accompanies the left coronary artery. The Posterior Coronary Plexus is chiefly formed by filaments prolonged from the left half of the deep part of the cardiac plexus, and by a few from the right half. The Posterior Coronary Plexus gives branches to the left atrium and ventricle.

The Anterior Coronary Plexus (plexus coronarius anterior; right coronary plexus) is formed partly from the superficial and partly from the deep parts of the cardiac plexus. The Anterior Coronary Plexus accompanies the right coronary artery. The Anterior Coronary Plexus gives branches to the right atrium and ventricle.

The pulmonary plexuses are the sites of convergence of autonomic fibres which supply the lung. The pulmonary plexuses are in continuity with the cardiac plexuses, which lie superiorly, and the oesophageal plexuses, which lie posterosuperiorly.

The pulmonary plexuses are sited anterior and posterior relative to each lung root. The pulmonary plexuses are in close proximity to the pulmonary arteries and, as they branch laterally, the pulmonary plexuses ramify their nerve fibres in association with the outer walls of diverging pulmonary arteries and arterioles.

The passage of fibres from the cardiac plexus is inferiorly, anterior to the trachea and posterior to the aortic arch. The pulmonary plexus also receives autonomic fibers directly from other sources. The pulmonary plexus receives parasympathetic fibers directly from the right vagus nerve, which descends posteroinferiorly on the trachea and divides posterior to the trachea to give pulmonary and oesophageal plexuses; pulmonary plexus passes anteriorly to root of the lung. The pulmonary plexus also receives parasympathetic fibers directly from the left vagus nerve, which descends anteriorly to arch of aorta, gives off recurrent laryngeal branch, and then fibres diverge anteriorly to supply the left pulmonary arterial plexus. The pulmonary plexus receives sympathetic fibers directly from rami of the superior four thoracic ganglia, which pass anteriorly around the posterior thoracic cage to merge on the lateral walls of the oesophagus. the rami supply nerve fibres to the pulmonary plexus from the region dorsal to the tracheal bifurcation.

The recurrent cardiac nerve and sometimes the craniovagal cardiac nerves can carry the main innervation of the pulmonary bifurcation and adjacent parts of the main pulmonary artery and its right and left branches. The recurrent cardiac nerve is a moderately large nerve, arising from the right recurrent laryngeal nerve as it loops around the right subclavian artery. The recurrent cardiac nerve usually receives a contribution of varying size from the vagal, parasympathetic trunk, and another from the stellate ganglion. The nerve passes dorsally to the anterior vena cava, laterally to the brachiocephalic artery and arch of the aorta, to the pulmonary bifurcation, to where it divides into anterolateral and posterolateral branches. The anterolateral branch tends to be smaller. The branches then tend to fan out over the anterior and posterior aspects of the main pulmonary artery and communicate with plexi around the right and left pulmonary arteries and the pretracheal plexus. Some fibres continue to the heart and the coronary plexi. During its course, it communicates freely with the craniovagal cardiac nerves.

The right vagal cardiac nerves arise from the right vagus trunk caudal to the origin of the right recurrent laryngeal nerve. They fall into two groups, the cranial and caudal vagal cardiac nerves. These vary in size, number, and course. Including some of the smaller divisions, he right vagal cardiac nerves supply branches or twigs to the right pulmonary artery plexus, the antero and posterolateral branches of the right recurrent cardiac nerve at the pulmonary bifurcation, and to the plexus formed by the ventral branch of the vagus, anterior to the pulmonary root, and then terminate in the atrial wall. Small twigs or branches, variable in size and position and sometimes absent, are supplied to the pre-tracheal plexus and the plexus around the right and left pulmonary artery by the right stellate cardiac nerves, the venteromedial cervical cardiac nerve, the left recurrent laryngeal nerve, and the ventral branch of the left vagal trunk. Other twigs or branches are supplied from a diffuse plexiform network of fibres form the ventrolateral cardiac nerve and the left stellate cardiac nerve.

One of these nerves that is of interest is the recurrent cardiac nerve, especially the right recurrent cardiac nerve, as it can contain pre-ganglionic, afferent and sympathetic post-ganglionic fibres among others. The recurrent cardiac nerve is a branch of the right recurrent laryngeal nerve, the nerve of visceral arch. It is therefore of considerable interest that the main nerve supply to the pulmonary bifurcation sensory area, part of the visceral arch, is derived from the recurrent laryngeal nerve, the nerve of visceral arch. As the most cephalic part of the pulmonary artery is formed from the posterior and right lateral parts of the bulbus cordis, this vessel is predominantly supplied from the right visceral nerve.

More specifically, the pulmonary artery bifurcation and adjacent portions of the right and left pulmonary arteries receive a very rich innervation. On the right side, the most constant nerve trunk to the bifurcation is the right recurrent cardiac nerve. The fibers arise from the vagus or the recurrent laryngeal nerve as it loops around the subclavian artery immediately cuadad to its origin from the brachiocephalic trunk. The nerve proceeds medially and caudally passing dorsal to the superior vena cava and lateral to the origin of the brachiocephalic trunk. The fibers ramify at the bifurcation by dividing into antero-lateral and postero-lateral branches which communicate with the fibers from the pulmonary plexuses. During its course it communicates with one or more right vagal cardiac nerves, usually of very small size, and branches from the stellate ganglia or ansa subclavia. These latter branches are thought to contribute the efferent component. Minor variation in the mode of origin from the recurrent laryngeal nerve (RLN) were noted. In some cases, the nerve can arise as a separate trunk from the loop of the RLN and can be joined by a cardiosympathetic branch from the adjacent stellate ganglion. The recurrent cardiac nerve can rarely arise from the angle of origin of the RLN as well. In some cases, the major portion of the nerve can arise from the vagus as the vagal cardiac nerve, also receiving a small filament from the RLN.

The contribution to the innervation of the pulmonary artery from the left side is similar to that of the right, but also receives in some cases invariably a small, direct contribution from the vagus in the form of the ventro-medial-cervical cardiac nerve. This nerve arises from the vagus by a variable number of roots, usually two, and proceeds caudally passing over the aortic arch to ramify over the ligamentum arteriosum, pulmonary bifurcation and left pulmonary artery. The superior cranio vagal root usually receives a direct branch from the left stellate ganglion. The bifurcation and left pulmonary artery receive a small inconstant branch from the RLN as it passes under the aortic arch. In some cases, the descending branches arise from the ascending portion of the RLN to terminate around the bifurcation.

The musculature of the pulmonary artery receives a right sided innervation of predominantly vasoconstrictor adrenergic sympathetic fibers, but little to no motor innervation from the parasympathetics or vagus nerve. The fibers synapse mainly in the stellate, but also in the upper thoracic and sympathetic ganglia. A large concentration of nerve endings are found at the bifurcation of the pulmonary artery, as well as in parts of the adjacent pulmonary artery and its right and left main branches.

Beyond the main pulmonary artery, right main and left main pulmonary arteries, the innervation of the further branches of the lung follows the arterial anatomy, with the nerves coursing along the arteries, typically following a peri-adventitial location or coursing along the adventitia. A rich innervation exists in pulmonary arteries further distal and to pulmonary arterioles as small as 30 microns in diameter or smaller. This innervation includes both parasympathetic and sympathetic innervation, with the lungs considered to have a rich sympathetic nerve supply.

Thoracic sympathectomy is a surgical procedure that currently exists and is utilized in the treatment of a different disease process, namely hyperhidrosis syndrome (excessive sweating). Extensive research on this surgical procedure has shown it to be safe and efficacious. Physiological studies of patients undergoing thoracic sympathectomy have shown mild changes in pulmonary function and mild increases in airway resistance, small decreases in heart rate however preserved left ventricular function and ejection fractions, and also preserved exercise tolerance. Data from T2-T3 video assisted thoracoscopic sympathectomy patients have shown that sympathectomy results in severing the ipsilateral hypoxia mediated vasoconstrictive pathway to the pulmonary vasculature by demonstrating a drop in arterial oxygen saturation during contralateral selective lung ventilation both prior and subsequent to sympathectomy. This implies ipsilateral pulmonary vascular dilatation and reduction in pulmonary pressure Although thoracic sympathectomy has been used for treating hyperhidrosis, it has not been described, prior to the provisional patent application from which this application claims priority, for treating pulmonary hypertension. More generally, decreasing activity of one or more sympathetic nerves or neurons to reduce pulmonary vascular resistance and/or to ameliorate pulmonary hypertension has not been described previously.

### Treatment Devices

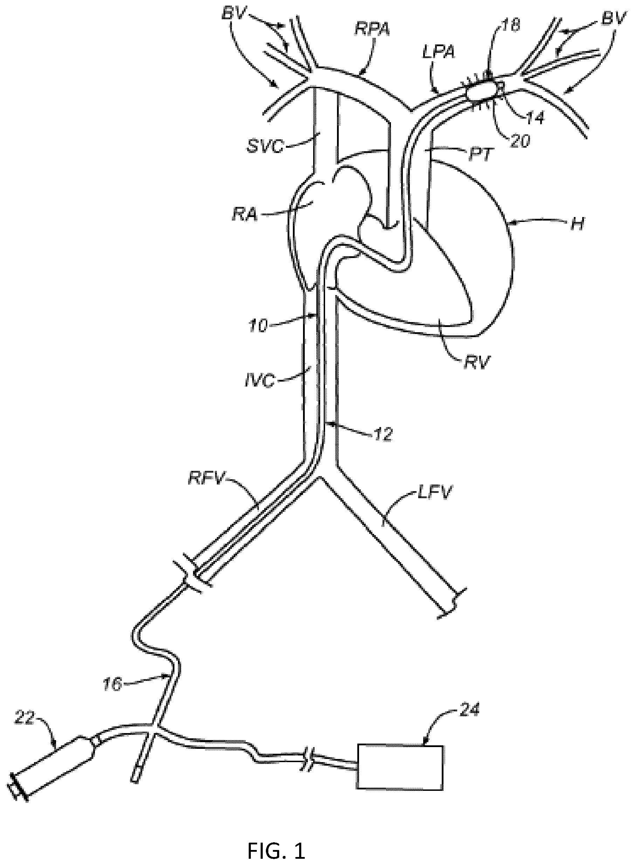

Fig. 1 schematically illustrates the general anatomy of the heart including pulmonary arteries and an example treatment catheter 10. The access pathway illustrated in Fig. 1 is one example of many possible access pathways for use with the catheter 10 or alternative catheters. In some embodiments, the catheter 10 is configured to decrease activity of one or more sympathetic nerves. The catheter 10 may include an elongate, flexible shaft 12 having a distal portion 14 and a proximal portion 16, an anchor 18 at or near the distal portion 14, a transducer (e.g., as described herein) at or near the distal portion 14, and an actuator 22 for deploying or collapsing the anchor 18.

The catheter 10 may comprise a plurality of lumens. For example, an optional lumen may be used to track the catheter 10 over a guidewire. For another example, an optional lumen may provide a passage for conductor wires between a transducer and a signal generating system. For another example, an optional lumen may provide passage for a conductor wire between a sensor and a receiving station.

As illustrated in Fig. 1, the shaft 12 can be advanced through an access point in a peripheral vessel, such as the right femoral vein RFV, into the inferior vena cava IVC, through the right atrium RA of the heart H, into the right ventricle RV, and then through the pulmonary trunk PT to the left pulmonary artery LPA. Other anatomical structures labeled in Fig. 1 include the right pulmonary artery RPA, branching vessels BV, superior vena cava SVC, and left femoral artery LFA. In some embodiments, the shaft 12 can be advanced through an access point in the LFV, into the inferior vena cava IVC, through the right atrium RA of the heart H, into the right ventricle RV, and then through the pulmonary trunk PT to the left pulmonary artery LPA. In certain such embodiments, the shaft can have a length between about 100 cm and about 150 cm (e.g., about 100 cm, about 110 cm, about 120 cm, about 130 cm, about 140 cm, about 150 cm, and ranges between such values). In some embodiments, the shaft 12 can be advanced through an access point in a jugular vein, ulnar vein, etc., into the SVC, through the right atrium RA of the heart H, into the right ventricle RV, and then through the pulmonary trunk PT to the left pulmonary artery LPA. In certain such embodiments, the shaft can have a length between about 60 cm and about 120 cm (e.g., about 60 cm, about 75 cm, about 90 cm, about 105 cm, about 120 cm, and ranges between such values). The shaft 12 is generally advanced through the vasculature and heart to a target location in the vasculature.

In some embodiments, a Swan-Ganz catheter may be inserted into the access point and floated to the target location. A guidewire can be advanced through the Swan-Ganz catheter, and the Swan-Ganz catheter can be removed, leaving the guidewire in place from the access point to the target location. The shaft 12 can be advanced over the guidewire. In some embodiments, the treatment catheter 10 can include features of a Swan-Ganz catheter such as a floatable balloon, and the treatment catheter 10 may be inserted into the access point and floated to the target location. In some embodiments, a guidewire can be steered, for example under fluoroscopy, from the access point to the target location. The shaft 12 can be advanced over the guidewire. Other positioning methods are also possible.

This target location may be any of a number of locations in various embodiments, such as but not limited to the pulmonary trunk PT, the left pulmonary artery LPA, the right pulmonary artery RPA, any of the branching vessels BV, the ostia of the left pulmonary artery LPA and/or right pulmonary artery RPA, and/or the like. In some embodiments, a different access method may be used, and a pulmonary vein or other pulmonary venous vasculature may be the target location. Additional access routes and potential targets are described in further detail herein.

Once at the target site, the device interrupts the nerves around the left, right, and/or main pulmonary arteries. In some embodiments, neuromodulation is accomplished (e.g., via ablation, denervation, which may or may not be reversible, stimulation, etc.), for example using acoustic energy (e.g., ultrasound), microwave energy, radiofrequency (RF) energy, thermal energy, electrical energy, infrared energy, laser energy, phototherapy, plasma energy, ionizing energy, mechanical energy, cryoablation, chemical energy, combinations thereof, and the like.

### Transducers

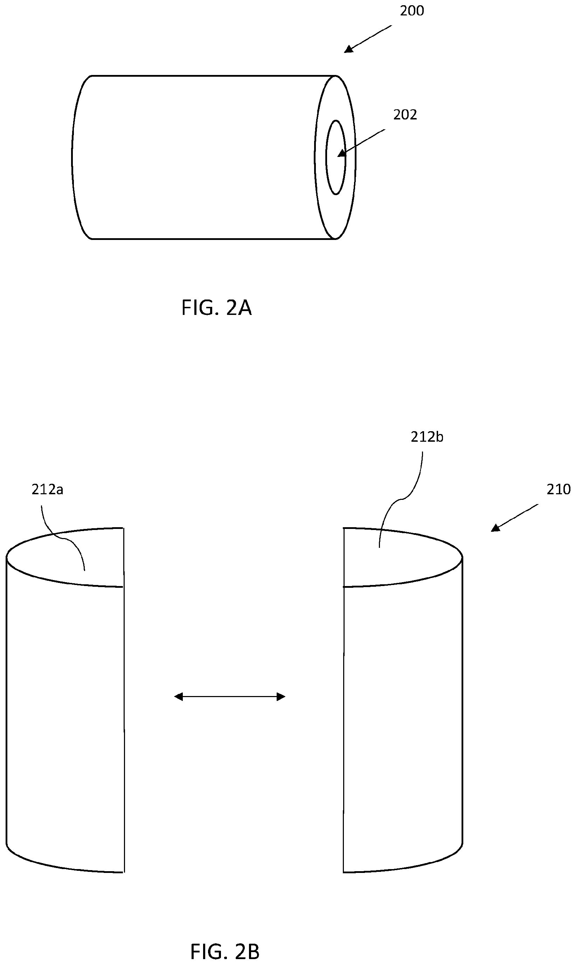

Fig. 2A illustrates an example transducer 200. The transducer 200 may be positioned at or near a distal portion of a catheter (e.g., the catheter 10). The transducer 200 may be separate from any anchor (e.g., as described herein). The transducer 200 may be coupled to a shaft (e.g., the flexible shaft 12). The transducer 200 may comprise a hole 202 extending therethrough, for example for coupling to a wire or tube of a catheter. Guidewires or sensor wires may extend through the hole 202. In some embodiments, the transducer 200 is an arcuate ultrasound transducer comprising a piezoelectric element.

The outer diameter of the transducer 200 may be between about 3 mm and about 10 mm (e.g., about 3 mm, about 4 mm, about 5 mm, about 6 mm, about 7 mm, about 8 mm, about 9 mm, about 10 mm, and ranges between such values). The transducer 200 may have a length between about 5 mm and about 30 mm (e.g., about 5 mm, about 10 mm, about 15 mm, about 20 mm, about 25 mm, about 30 mm, and ranges between such values). A longer and/or thicker transducer 200 can generally provide more power. A shorter and/or thinner transducer 200 may be easier to navigate through vasculature. A thinner transducer 200 may be used with a smaller incision, which can reduce scar size, infection site size, and/or healing time. A ratio between the diameter of the transducer 200 and the length of the transducer 200 may be between about 1/20 and about 2/1 (e.g., about 1/20, about 1/15, about 1/10, about 1/5, about 1/3, about 1/1, about 3/2, about 2/1, and ranges between such values).

Fig. 2B illustrates another example transducer 210. The transducer 210 may comprise a first hemicylinder 212a and a second hemicylinder 212b. The first hemicylinder 212a and the second hemicylinder 212b may be coupled to form a cylindrical shape. The first hemicylinder 212a may be activated for ablation while the second hemicylinder 212b is inactive. Partial activation can provide partial circumferential ablation, for example to protect sensitive structures in the area around the second hemicylinder 212b. The first hemicylinder 212a may be activated for ablation and the second hemicylinder 212b may be activated for ablation. Coordinated activation can provide full circumferential ablation, for example to treat tissue all around a vessel. Full circumferential ablation, as can be provided by the transducer assemblies provided herein, can reduce or eliminate rotation at an ablation site. In some embodiments, the transducer 210 is an arcuate ultrasound transducer comprising a piezoelectric element.

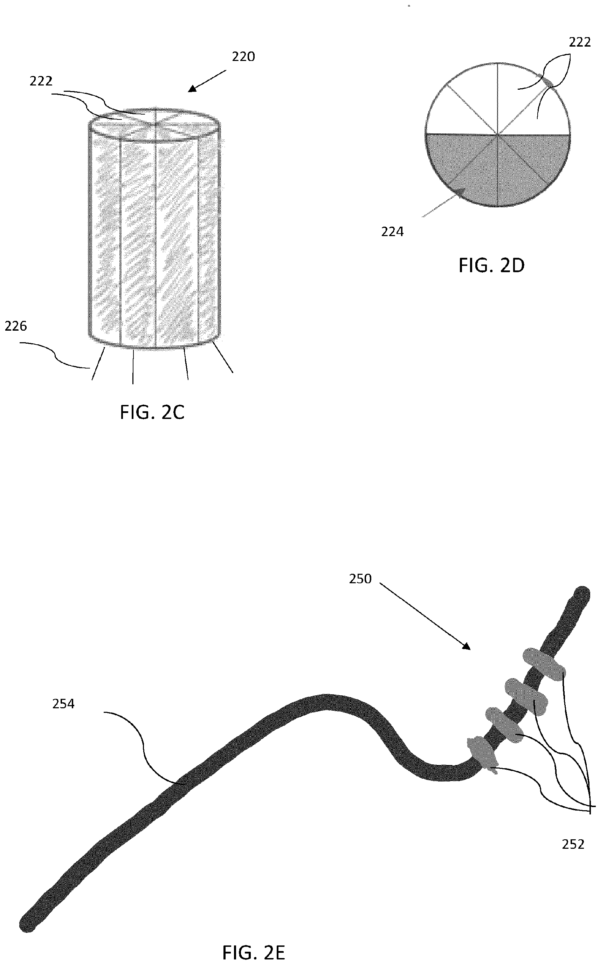

Fig. 2C illustrates another example transducer 220. A transducer 220 may comprise a plurality of angular or wedge-shaped arcuate regions 222. Eight angular regions 222 are depicted in Fig. 2C, but any numbers of regions may be used (e.g., two regions (e.g., as shown in Fig. 2B), three regions, four regions, five regions, six regions, seven regions, eight regions (e.g., as shown in Fig. 2C), nine regions, ten regions, eleven regions, twelve regions, and ranges between these values). The regions 222 can all be the same. At least one of the regions 222 can be different than at least one other of the regions 222. For example, a difference may include a material, a shape, a dimension, and/or the like.

The angular regions 222 may be activated through a plurality wires 226 each connected to an ultrasound system and to one or more of the regions 222. In some embodiments, the user can decide which regions 222 to activate during ablation. For example, the angular regions 222 in Fig. 2C that are shaded are activated for ablation while the angular regions unshaded are not activated for ablation. Regions 222 facing sensitive structures may not be activated to preserve those sensitive structures from being ablated. A larger quantity of the regions 222 can provide more activation flexibility in certain such embodiments. A smaller quantity of the regions 222 can provide less manufacturing complexity. In some embodiments, each angular region 222 comprises a spring contact pad that can allow electrical power to flow through the disc when the disc is over the region 222.

Fig. 2D illustrates an example transducer connection implementation in which stacked discs 224 that rotate on the edge of the transducer 220 can electrically connect or disconnect the angular regions 222. As shown in Fig. 2D, the angular regions 222 that are shaded are electrically connected via the stacked discs 224 and can be collectively activated for ablation. The angular regions 222 are not connected via the stacked discs 224 cannot be activated for ablation. In some embodiments, the stacked discs 224 may be rotated independently through push and pull wires. In some embodiments, the stacked discs 224 comprises a single half disc 224, two stacked half discs 224, a single two-thirds disc 224, a single three-quarters disc 224, etc. The discs 224 can provide an ablation profile that inhibits or prevents ablation in a region where a sensitive structure or other structure desirably not ablated is located. The rotation of the disc 224 can be controlled from the proximal side of the catheter, for example through a wheel that rotates a shaft with appropriate torquability. Two radiopaque symbols located on the actuating shaft can inform the operator about the positioning of the discs 224 and thus about the ablation profile that is or would be created.

Referring again to Fig. 1, the catheter 10 may need to navigate somewhat tortuous anatomy, including, for example, a right turn into the right atrium followed soon by a U-turn in the right ventricle. If any portion of the catheter 10 is too stiff or not flexible enough to make such turns, then the distal portion 14 might not be able to be delivered to the target location(s). Fig. 2E schematically illustrates an example transducer 250 comprising a plurality of longitudinal segments 252. The transducer 250 may comprise any suitable quantity of longitudinal segments 252 (e.g., two segments, three segments, four segments (e.g., as shown in Fig. 2E), five segments, six segments, and ranges between such quantities). More segments 252 are also possible. The segments 252 can be abutting or spaced apart by a distance. The segments 252 may be spaced during navigation and then an actuator (e.g., controlling a pull wire and/or push rod) can cause the segments to abut during ablation. The transducer 250 can ease the bending of the distal portion of the catheter during navigation to the target location(s) because the catheter is able to bend between the segments 252. In some embodiments, the segments 252 can provide electronic focusing of an ultrasound beam using phased wave generation. The segments 252 may be partially activated, for example similar to the hemicylinder portions 212a, 212b and/or angular regions 222 described herein. For example, one, some, or all of the segments 252 may be activated for ablation depending on where the targeted nerves for ablation are located and where sensitive structures may be located.

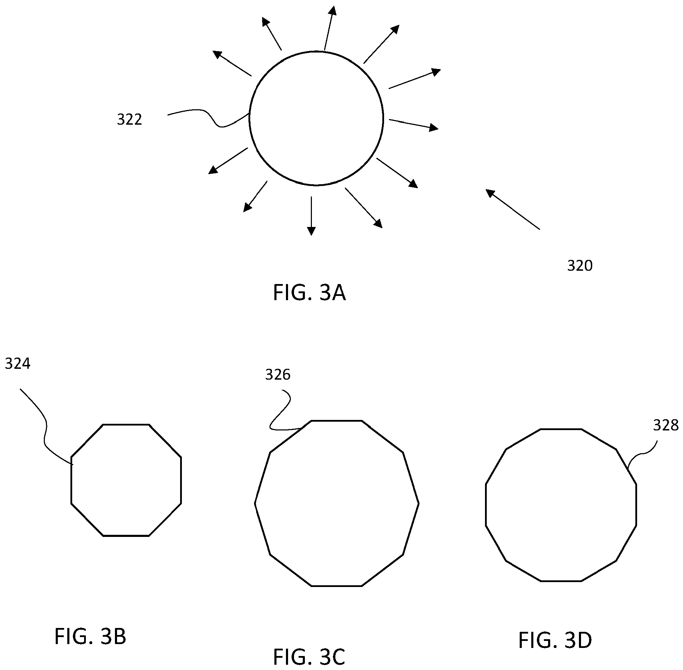

Figs. 3A-3D illustrate example outer surface shapes of example transducers. Fig. 3A is an end view or a cross-sectional view of a transducer 320 having a round outer shape 322. The outer surface of the transducer 320 does not need to be a perfect circle. For example, the transducer 320 may be oval, elliptical, egg-shaped, etc. The transducer 320 having an outer surface shape being round or arcuate can provide an ultrasound beam that is projected out in all directions, for example as schematically shown in Fig. 3A. An ultrasound beam projecting out in all directions can allow for ablation to occur around all areas surrounding a vessel wall at an ablation site, which optionally reduces or eliminates rotation of the transducer 320 because an entire circumferential area can be treated with one ablation. Using only one ablation can reduce procedure time. Reduced procedure time for a target location can be especially important, for example, when multiple target locations are treated (e.g., multiple locations in the RPA, LPA, and PT), and/or if an anchor is collapsed and then re-expanded between ablations.

Figs. 3B-3D illustrate additional example outer surface shapes of transducers. Fig. 3B is an end view or a cross-sectional view of a transducer 324 having an octagonal outer shape. Fig. 3C is an end view or a cross-sectional view of a transducer 326 having a decagonal outer shape. Fig. 3D is an end view or a cross-sectional view of a transducer 328 having a dodecagonal outer shape. Transducers having any number of polygonal sides, preferably greater than five and less than 32, are also possible. While not a perfect circle, a higher number polygon outer shape can function similarly to a round outer shape in that the ultrasound beams projected out are in all directions from the transducer and produce a large amount of coverage. The transducer does not have an overall flat shape. For example, the transducer is not flat, two-sided, triangular, square, trapezoidal, parallelogram, or rectangular. Transducers of flat shapes, such as those listed herein, may not be able to provide a complete projection of ultrasound energy, and/or may require rotating the transducer to ablate all targeted nerves.

The transducers 322, 324, 326, 328 may include a plurality of hemi-pieces or wedge-shaped regions (e.g., as described with respect to Figs. 2B and/or 2C), a plurality of longitudinal segments (e.g., as described with respect to Fig. 2E). Such regions and/or segments may be individually, partially, and/or collectively activated, as desired.

In some embodiments, the transducer may be configured to effect neuromodulation (e.g., via ablation, denervation, which may or may not be reversible, stimulation, etc.), for example by applying microwave energy, radiofrequency (RF) energy, thermal energy, electrical energy, infrared energy, laser energy, phototherapy, plasma energy, ionizing energy, mechanical energy, cryoablation, chemical energy, combinations thereof, and the like.

### Lenses

Any one of the transducers described herein or other transducers may optionally be coupled to a lens to focus or defocus the ultrasound energy. For example, energy from a cylindrical transducer can be focused by a lens to produce a toroid or doughnut-shaped treatment region around the transducer. Other shapes are also possible (e.g., spherical, ellipse, egg, arch, hemisphere, cigar, disk, plate, bulged versions thereof, etc.). The transducer may be acoustically coupled to the lens with piezoelectric material. The combination of the transducer and the lens may be called a transducer assembly, which may include the coupling material. In certain embodiments in which the device does not include a lens, the reference to a transducer assembly herein may refer to the transducer itself and optionally related components such as a conductor wire, material to couple the transducer to the shaft, etc. The focal length may be affected by the profile of the lens, the energy applied to the transducer, the efficiency of the components and/or assembly, and/or other parameters. In some embodiments, the efficiency of the assembly is tested by the manufacturer or a testing laboratory and the known efficiency is used during treatment.

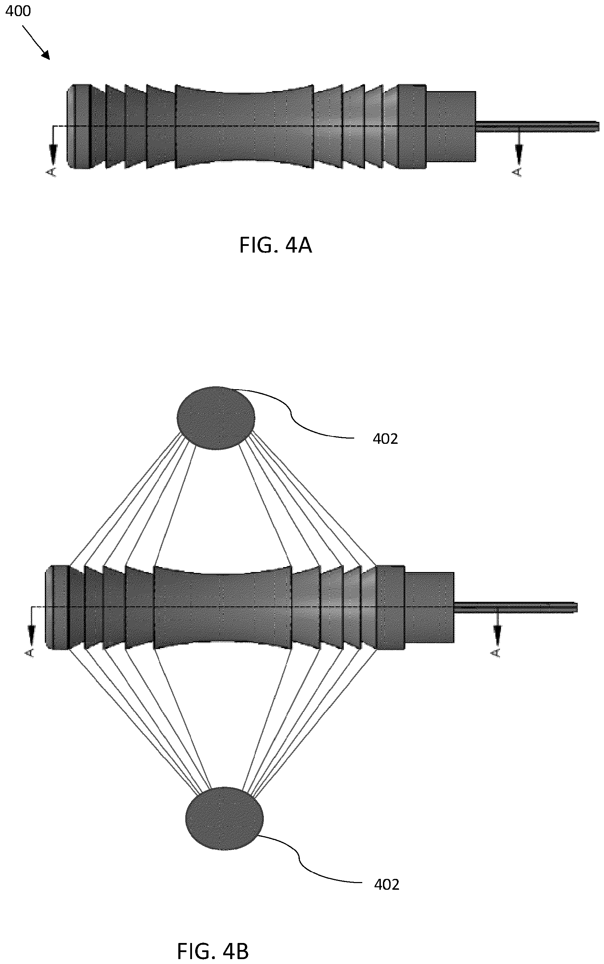

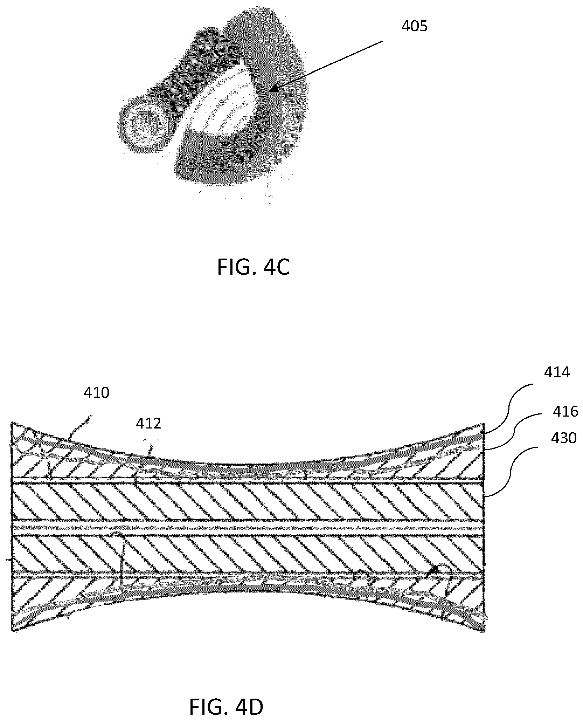

Fig. 4A illustrates an example Fresnel lens 400. The lens 400 is acoustically coupled to a transducer. The Fresnel lens 400 comprises a plurality of prisms configured to change the direction of the acoustic energy from the transducer so that the energy is generally focused to a common longitudinal area. The Fresnel lens can reduce the overall diameter of a catheter (e.g., catheter 10)and/or a portion thereof (e.g., a distal portion) because the prisms are able to redirect energy while maintaining a low profile (e.g., compared to a convex surface that may have a diameter that increases towards the longitudinal edges). Fig. 4B schematically illustrates example energy rays emanating from the prisms to longitudinally focus and concentrate acoustic energy to a smaller section 402 around the transducer assembly. In practice, the section 402 can be tissue surrounding a vessel in which the transducer is positioned. In some embodiments, for a 22 mm focal point measured from the center of the transducer radially outward, a lesion that can be created by the energy may be pre-focal (e.g., about 1.5-5 mm from the vessel wall, depending on vessel diameter and other parameters). A larger amount of energy delivered and/or a longer delivery duration can increase the focal depth. A smaller amount of energy delivered and/or a shorter delivery duration can reduce the focal depth. The focus point of the energy does not have to be a pinpoint location or band. Fig. 4C illustrates an example portion of an energy application shape. Part of a toroidal area 405 is illustrated. The energy application shape may be a full toroid, but shown in Fig. 4C as only part of a toroid for clarity. In some embodiments, the partial toroid shown in Fig. 4C may be created, for example, by activation of less than an entire transducer (e.g., one, two, or some wedges). The energy produced by a transducer may be at least partially absorbed by tissue in and/or around the vessel, which can create the toroidal ablation site 405. Other energy application shapes are also possible (e.g., spherical, ellipse, egg, arch, hemisphere, cigar, disk, plate, bulged versions thereof, etc.).

The lens preferably comprises one or more materials that are acoustically conductive, good thermal conductors, good electrical insulators, and/or biocompatible. No one material may possess all of these properties, so a plurality of layers can be used (e.g., the outer-most layer can be biocompatible to protect the body from inner layers that are not as biocompatible). In some embodiments, the lens comprises aluminum that has been anodized or otherwise treated to have a coating of aluminum oxide (alumina). The aluminum and alumina are both good thermal conductors, the aluminum is acoustically conductive (e.g., the speed of sound through aluminum is about 4 × the speed of sound through blood), and the alumina is biocompatible and a good electrical insulator. In some embodiments, the lens comprises silicon dioxide. Silicon dioxide is a good thermal conductor and biocompatible, and with certain doping, for example, may be suitably acoustically conductive.

Fig. 4D illustrates an example transducer assembly. The assembly includes another example of a lens 410 coupled to a transducer (e.g., as described herein). The lens 410 may be an ultrasonic lens. Fig. 4D is a cross-sectional view through the longitudinal axis L and depicts a transducer assembly comprising a cylindrical transducer 430 and an ultrasonic lens 410. The lens 410 has an inner cylindrical surface and an outer surface shaped with a concave profile. The lens 410 is acoustically coupled to the transducer 430. The transducer assembly also comprises a piezoelectric element 412. The lens 410 can focus energy from the transducer 430 (e.g., as described herein). Because the lens 410 does not include a plurality of prisms, the lens 410 may have a larger diameter than the Fresnel lens 400. The lens 410 may be easier to manufacture than the Fresnel lens 400. The lens 410 may be easier to flush with saline prior to insertion into vasculature than the Fresnel lens 400.

The lens 410, or the Fresnel lens 400, increases the surface in contact with blood inside the vessel, which can improve the ability of the transducer to cool down by acting as a heat sink. To act as a heat sink, the lens material is preferably a thermal conductor (e.g., aluminum, alumina, silicon dioxide). The plurality of prisms of the Fresnel lens 400 can act as fins for the heat sink. In some embodiments, the lens 400, 410 comprises a biocompatible layer 414. The lens covers the piezoelectric material 412 of the transducer 430 and inhibits or prevents contact between the blood and the outer surface of the transducer. In some embodiments, the lens comprises an electrical insulation layer 416. The insulation layer 416 isolates the patient from the high voltage used to drive the transducer energy. The lens material may support dielectric properties to protect the patient from high voltage.

The devices described herein may lack or be devoid of a cooling system, which can advantageously significantly reduce device cost. For example, the blood flow through the pulmonary arteries may be sufficient to cool the transducer assembly. In contrast, a transducer assembly positioned in a renal artery may not be exposed to sufficient blood flow to provide enough cooling, and such devices may include a cooling system (e.g., a saline lumen pumped through the transducer before, during, and/or after ablation).

The size of a lens may depend, for example, at least partially on the material(s) and/or frequency (e.g., the natural frequency and/or the applied frequency from the ultrasound beam generator). Frequency adjustments can be made during the calibration or the setup of the transducer, for example so such adjustments do not need to be made during a procedure. Different frequencies may be used to ablate different depths outside the vessel. The material selected for the lens may impact the frequency needed for ablation. For example, if an acoustically poor material such as glass is used, the lens would be thinner to account for the losses caused by the acoustically poor material. If, for example, the material used has good acoustics, the lens may be thinner. For example, for a 25 mm focal length at 3 MHz over a 4 mm outer diameter transducer, an aluminum lens (c = 6500 m/s) can have a profile of 5.4 mm, while an epoxy lens (c = 2430 m/s) can have a profile of 7 mm for the same focal length.

Each transducer and lens combination has an associated data sheet that characterizes the transducer assembly and accounts for the differences in the transducer and lens combinations. Since the absorption of the acoustic energy by the tissue is a function of the frequency of the ultrasound beam, the transducer assembly should be carefully designed to meet the desired specifications. In an example implementation, a 4 mm outer diameter transducer is coupled to a 5 mm outer diameter aluminum Fresnel lens having a 25 mm focal length for operation at 4.5 MHz. In another example implementation, a 4 mm outer diameter transducer is coupled to a 6 mm outer diameter epoxy Fresnel lens having a 25 mm focal length for operation at 4.5 MHz. In another example implementation, a 1.5 mm outer diameter transducer is coupled to a 2.15 mm outer diameter aluminum Fresnel lens having a 10 mm focal length for operation at 6 MHz. In another example implementation, a 1.5 mm outer diameter transducer is coupled to a 2.8 mm outer diameter epoxy Fresnel lens having a 10 mm focal length for operation at 6 MHz. The catheter may comprise one or a plurality of flushing ports to inhibit or prevent introducing bubbles inside the patient (e.g., bubbles that might otherwise be trapped in the prisms of a Fresnel lens).

### Anchors

During ablation, a transducer assembly (e.g., as described herein) may be anchored within a vessel. If the transducer assembly is not anchored, it may float or flop around in the blood flow, especially high blood flow like in pulmonary arteries, which can cause very unpredictable, or at the very least blurry and inefficient, ablation. In some embodiments, the position of the transducer can be steadied by an anchor.

The anchor may be configured to preserve blood flow through the vessel, including when the anchor is in a deployed state. A method including the anchor may comprise allowing blood to flow through the vessel when the anchor is in a deployed state. In some embodiments, the anchor does not comprise a balloon. For example, the edges of the prisms of a lens (e.g., the Fresnel lens 400) may damage a balloon anchor. In some embodiments, the anchor is not occlusive, allowing blood to continue to flow to downstream vessels and organs (e.g., the lungs). Renal denervation devices, for example comprising balloons, are typically occlusive because it is possible to pause blood flow to the kidneys without negative systemic effects. In some embodiments, a device configured to be used in pulmonary branch vessels (RPA and/or LPA) may comprise and anchor that occludes blood flow to one lung at a time because the other lung may be sufficient to oxygenate the blood for a short duration.

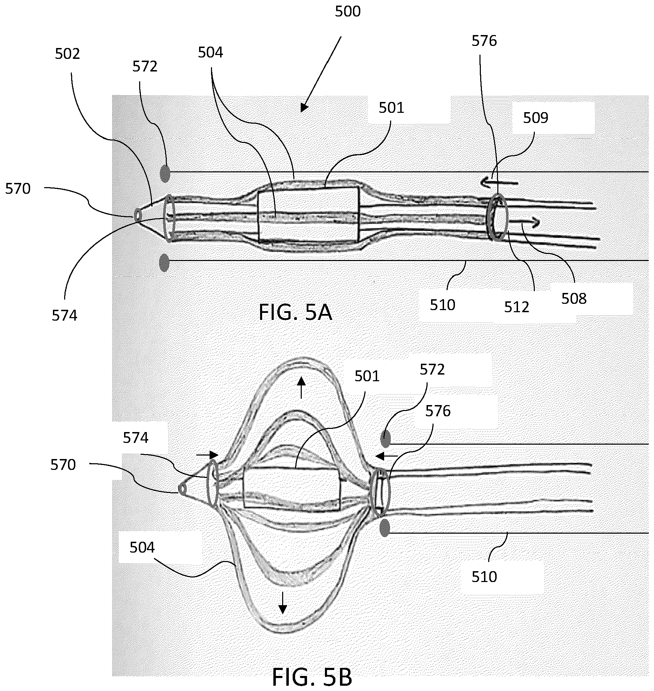

Fig. 5A illustrates an example embodiment of an anchor 500 comprising a plurality of struts 504 in a collapsed or delivery state. The anchor 500 is navigated to a vessel in the delivery or collapsed state. In some embodiments, the anchor 500 is optionally covered with a sheath during delivery or at other times in the delivery state. The anchor 500 is deployable towards a deployed state.

Fig. 5B illustrates the anchor 500 in the deployed state. Depending on the diameter of the vessel, it may not be possible to achieve the delivery state shown in Fig. 5B, but expansion of the anchor 500 such that the anchor 500 is able to maintain a substantially constant position of the transducer assembly 501 in the vessel may be considered the deployed state. Ablation preferably occurs when the anchor 500 is in the deployed state, or is not in the delivery state.

The plurality of struts 504 may, for example, be cut (e.g., laser cut) from a hypotube or sheet. Cutting the struts 504 from a tube or sheet may, for example, provide quick and repeatable manufacturing.

In some embodiments, the plurality of struts 504 are discrete wires. The wires are optionally not cut from a tube or sheet, or may be originally cut from a tube or a sheet in a manner that allows at least some of the struts 504 to be discrete (e.g., not directly coupled by strut material to another strut). Using discrete wires can provide flexibility in determining the shape and configuration of the struts 504. For example, the plurality of struts 504 may comprise wires that are straight, twisted, flat, round, combinations thereof, etc. (e.g., as shown in Figs. 5A-5D). The wires may have a polygonal cross-section (e.g., rectangle, square, diamond, trapezoid, bulged versions thereof, etc.), a round or arcuate cross-section (e.g., circle, oval, ellipse, etc.), combinations thereof, and the like.

The struts 504 may be coupled (e.g., individually coupled) (e.g., adhered, soldered, welded, not separated when being cut from a tube or sheet, combinations thereof, and the like) distal and proximal to the transducer assembly 501. As shown in Fig. 5A, distal portions of the struts 504 are coupled to a distal shaft 502 and proximal portions of the struts 504 are coupled to proximal shaft 512. The distal shaft 502 may comprise an atraumatic tip. The distal portions of the struts 504 may be coupled to the distal shaft 502 distal to the transducer assembly 501. The proximal portions of the struts 504 may be coupled to the proximal shaft 512 proximal to the transducer assembly 501. The transducer assembly 501 may be substantially radially centered between the struts 504 in the delivery state and/or in the deployed state. The distal shaft 502 is longitudinally movable relative to the proximal shaft 512. Such longitudinal movement may be allowed during self-expansion of the anchor 500 and/or used to expand the anchor 500. When the struts 504 bow radially outward, the longitudinal distance between the distal portion of the struts 504 and the proximal portion of the struts 504 is reduced. The transducer assembly 501 may be substantially radially centered between the struts 504 in the delivery state and/or in the deployed state.

In some embodiments, the anchor 500 is deployed by pushing the proximal and distal portions of the struts 504 together (e.g., proximally retracting the distal shaft 502 and/or distally advancing the proximal shaft 512), causing the struts 504 to bow radially outwards, as shown in Fig. 5B. In the deployed state, the plurality of struts 504 expand out to contact or appose vessel walls. The anchor 500 may maintain a longitudinal position of the transducer assembly 501. This umbrella-type method of deployment can provide better control of the radial force being applied to the vessel wall by the anchor 500. If the movement is manual by a hand of a user, for example, the user will be able to feel when the struts 504 contact the vessel wall and stop expanding the struts 504 at the appropriate deployed state. If the movement is motorized, for example, sensors may be used to measure force and stop movement upon reaching a certain force. To return to a delivery state 500, the struts 504 are pulled apart (e.g., by distally advancing the distal shaft 502 and/or proximally retracting the proximal shaft 512) to collapse the struts 504 back to the delivery state. The anchor 500 is configured to expand to fit any appropriate size vessel. For example, the LPA, RPA, and PT do not have the same diameters as each other or uniform intra-vessel diameters, and the anchor 500 is configured to expand to contact the vessel wall in all suitable locations of the LPA, RPA, and PT. In implementations such as ablation around renal arteries, the anchor 500 is configured to expand to contact the vessel walls in all suitable locations in the left renal artery and the right renal artery.

The struts 504 may be self-expanding. For example, the anchor 500 may be collapsed and deployed by retracting and advancing an outer sheath 510 to expose or cover the struts 504. The outer sheath 510 is proximally retracted in the direction of the arrow 508 to allow the struts 504 to at least partially self-expand. The anchor 500 is returned to the collapsed state by distally advancing the outer sheath 510 in the direction of the arrow 509 to apply a radially inward force to the struts 504 to cause the struts 504 to collapse. In some embodiments, the outer sheath 510 may be distally advanced to deploy the struts 504 and proximally retracted to collapse the struts (e.g., using a push-pull mechanism such as a pull wire extending through the distal portion 502).

In some embodiments, the outer sheath 510 may be used with umbrella-type expansion. For example, the outer sheath 510 can protect the vasculature from the struts 504 and vice versa during navigation to the target location. For another example, the outer sheath 510 can have a lubricious surface that aids in navigation. For another example, the outer sheath 510 can hold one or more sensors useful for measuring parameters near the transducer assembly 501. For another example, the outer sheath can comprise a Swan-Ganz balloon to float the catheter to a target location (without using a separate Swan-Ganz catheter).

The outer diameter of the distal portion of the catheter including the transducer assembly 501, the anchor 500, and optionally the outer sheath 510 is between about 3 mm and about 12 mm (e.g., about 3 mm, about 4 mm, about 5 mm, about 6 mm, about 7 mm, about 8 mm, about 10 mm, about 12 mm, and ranges between such values). A smaller diameter distal portion can allow insertion through a smaller incision. A smaller incision can reduce scar size, potential infection site size, and/or healing time.

In some embodiments, a combination of partial self-expansion and umbrella-type expansion are used. For example, the outer sheath 510 may be proximally retracted, which can allow the struts 504 to partially self-expand. This partial self-expansion may be sufficient to appose the vessel walls. In some alternative implementations in which anchoring is not desired but spacing between the transducer assembly 501 and the vessel wall can be provided by the struts being partially self-expanded, this partial self-expansion may be sufficient. If the partial self-expansion is not sufficient (e.g., to sufficiently appose the vessel walls), then the umbrella-type expansion may be used to further expand the struts 504, for example as described herein.

The plurality of struts 504 preferably comprise a shape-memory material (e.g., nitinol, chromium cobalt, MP35N, 35NPT, elgiloy, etc.). Even in embodiments in which the anchor 500 is not purely self-expanding, shape-memory material can help the plurality of struts 504 maintain a shape, respond to external forces (including device-based expansion forces), etc. Other strut materials are also possible (e.g., stainless steel).

In some embodiments, the struts 504 are not aligned with the transducer. For example, even if the transducer comprises four wedge-shaped pieces and the anchor 500 comprises four struts 504, the struts 504 do not necessarily need to be aligned with (e.g., at intersections of) the transducer pieces. Rather, the struts 504 can be independent of the transducer pieces.

The transducer assembly 501 may be substantially radially centered between the struts 504. If the struts uniformly expand, then the transducer assembly 501 may be substantially centered in the vessel. Centering the transducer assembly 501 in the vessel can help ensure that tissue all around the vessel is treated. For example, if the transducer assembly 501 has a penetration radius of 20 mm and is centered in a vessel where the diameter of the vessel is 18 mm, then the penetration depth all around the vessel is about 11 mm. If the same transducer assembly 501 was not centered in that same vessel, then penetration depth could be 3 mm in one direction and 19 mm in the opposite direction, either or both of which could affect undesired tissue. It will be appreciated that these numbers are for example purposes and that true numbers would take into account, for example, ultrasound absorption, diffraction at interfaces, Snell Descartes' law, etc.

Figs. 5A and 5B schematically illustrate positions of some example radiopaque markers 570, 572, 574, 576. The marker 570 is at a distal tip of the distal portion. The marker 570 may be slightly spaced from the distal tip of the distal portion. The marker 572 is at a distal end of the outer sheath 510. The marker 572 may be slightly spaced from the distal end of the outer sheath 510. The marker 574 is at a distal end of the anchor 500. The marker 574 may be slightly spaced from the distal end of the anchor 500. The marker 576 is at a proximal end of the anchor 500. The marker 576 may be slightly spaced from the proximal end of the anchor 500. In some embodiments, the material of the anchor 500 may be radiopaque such that the marker 574 and/or the marker 576 is the visible ends of the anchor 500 (e.g., no separate marker material is used).

The marker 570 can be used to control the distal tip of the device, for example to inhibit or prevent perforation distal to the treatment site and/or to inhibit or prevent application of pressure on small vessels. The marker 572 can be used to determine the position of the outer sheath 510, for example relative to other components. If the marker 572 is distal to the marker 574, the user knows that the anchor 500 is covered by the outer sheath 510. If the marker 572 is proximal to the marker 576, the user knows that the anchor 500 is not covered by the outer sheath 510. The user may observe the relative positions of the markers 574, 576 to gauge the expansion of the anchor 500. For example, as seen in Figs. 5A and 5B, when the markers 574, 576 are further apart, the anchor 500 is closer to the collapsed position, and when the markers 574, 576 are closer together, the anchor 500 is closer to the deployed or expanded position. In some embodiments, the distance between the markers 574, 576 can be measured (e.g., directly using fluoroscopy measurement (e.g., using the length of the transducer 501 for scale), using indicia on the device, etc.) to determine the extent of expansion, which may include the diameter of the vessel at the deployment site. The extent of expansion and/or the diameter of the vessel at the deployment site may be used to set neuromodulation (e.g., ablation) parameters.

The transducer assembly 501 may be longitudinally moveable relative to the distal shaft 502 and/or the proximal shaft 512 (e.g., by being coupled to an independent transducer shaft). For example, although the transducer assembly 501 is illustrated as being large relative to the anchor 500, the transducer assembly 501 could extend over a much smaller longitudinal extent of the anchor 500. The transducer assembly 501 could move relative to the anchor 500 to perform a plurality of ablations without collapsing and redeploying the anchor. For example, the transducer assembly 501 could be at a first distal position in the anchor 500, perform a first ablation, then can be proximally retracted to a second intermediate position in the anchor 500 without moving the anchor 500, perform a second ablation, then can be further proximally retracted to a third proximal position in the anchor 500 without moving the anchor 500, and perform a third ablation. In some embodiments, the transducer assembly 501 could be at a first proximal position in the anchor 500, perform a first ablation, then can be distally advanced to a second intermediate position in the anchor 500 without moving the anchor 500, perform a second ablation, then can be further distally advanced to a third distal position in the anchor 500 without moving the anchor 500, and perform a third ablation. In some embodiments, the transducer assembly 501 could be at a first intermediate position in the anchor 500, perform a first ablation, then can be distally advanced to a second distal position in the anchor 500 without moving the anchor 500, perform a second ablation, then can be proximally retracted to a third proximal position in the anchor 500 without moving the anchor 500, and perform a third ablation. The movability of the transducer assembly 501 in the anchor 500 is generally more important than the precise implementation of movement. While this may be mechanically more complex (e.g., as opposed to the transducer assembly 501 being mounted between the distal shaft 502 and the proximal shaft 512), such movement could reduce operation time by reducing or eliminating the collapsing, repositioning, and redeploying of the anchor after each of the first and second ablations.

The struts 504 may have a thickness between about 30 µm and about 500 µm (e.g., about 30 µm, about 40 µm, about 50 µm, about 60 µm, about 70 µm, about 80 µm, about 90 µm, about 100 µm, about 110 µm, about 120 µm, about 130 µm, about 140 µm, about 150 µm, about 500 µm, and any ranges between these values). This thickness is measured in the radial direction of each individual strut 504. The thinner the struts 504, the less likely the struts 504 are to cause interference or scattering with an ultrasound signal. For example, the struts 504 may cast an ultrasound shadow resulting in areas covered by the shadow not being ablated.

The plurality of struts 504 may comprise between about four struts and about 64 struts (e.g., about four struts, about six struts, about eight struts, about ten struts, about twelve struts, about sixteen struts, about twenty struts, about thirty struts, about forty struts, about fifty struts, about 64 struts, and ranges between such values).

The applicant has discovered that strut thicknesses less than about 100 µm does not appreciably affect an ultrasound signal. In embodiments having thin struts 500 (e.g., between about 30 µm and about 100 µm), a larger quantity of struts (e.g., between about ) may be used to increase the amount of total apposition force on the vessel wall to provide suitable anchoring.

Some embodiments may comprise thicker struts 504 (e.g., between about 110 µm and about 500 µm). For example, the interference or shadow caused by the struts 504 may be advantageously used to protect a portion of the vessel wall while ablating the targeted tissue (e.g., including nerves) beyond the vessel wall. Thicker struts 504 may provide higher radial force on the vessel wall for a more secure anchoring.

A balance between reducing interference or a shadow produced by the struts 504 and sufficient radial force may be desirable. The number or quantity of struts 504 may be varied to counteract any interference or shadows and/or to increase radial force as may be appropriate. A lower number of struts 504 can reduce potential interference and shadows. A higher number of struts 504 can increase radial force.

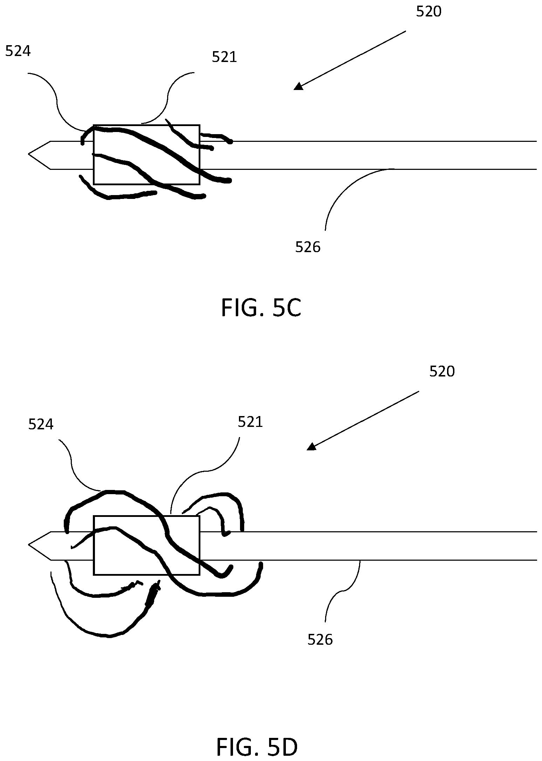

Fig. 5C illustrates another example of an anchor 520. Fig. 5C illustrates a collapsed or delivery state of the anchor 520. Fig. 5D illustrates a deployed state of the anchor 520. The anchor 520 comprises a plurality of struts 524 that are twisted around the transducer 521. The plurality of struts 524 may be collapsed and deployed via any of the methods described herein (e.g., self-expanding, umbrella-type, and combinations thereof). The twisted configuration of the plurality of struts 524 can reduce the overall interference or ultrasound shadows that the plurality of struts 524 may create across the transducer assembly 521. For example, there is less interference produced in the longitudinal direction by each strut 524 because the twisted configuration will only cover a portion of the transducer assembly 521 in the longitudinal direction instead of an entire section of the transducer assembly 521 in the longitudinal direction.

As shown in Fig. 5D, along any longitudinal line, there may be a portion of one or several struts 524, but there is no longitudinal line that is entirely a strut. The twisted configuration allows for coverage by the struts 524 to be positioned in various sections of the transducer assembly 521, such that entire longitudinal sections are not covered. Combined with the application of power to the length of the transducer assembly 521 and the focusing provided by the lens, a twisted configuration of the plurality of struts 524 may increase the probability of all the targeted tissue (e.g., including nerves) being ablated due to the reduction in potential interference or shadows caused by the struts 524. Twisted struts 524 can provide a partially lateral dimension to the anchor 520, which can help to provide better vessel wall apposition, for example providing a counter force to longitudinal blood flow. Straight struts 504 may be less prone to cause blood turbulence.

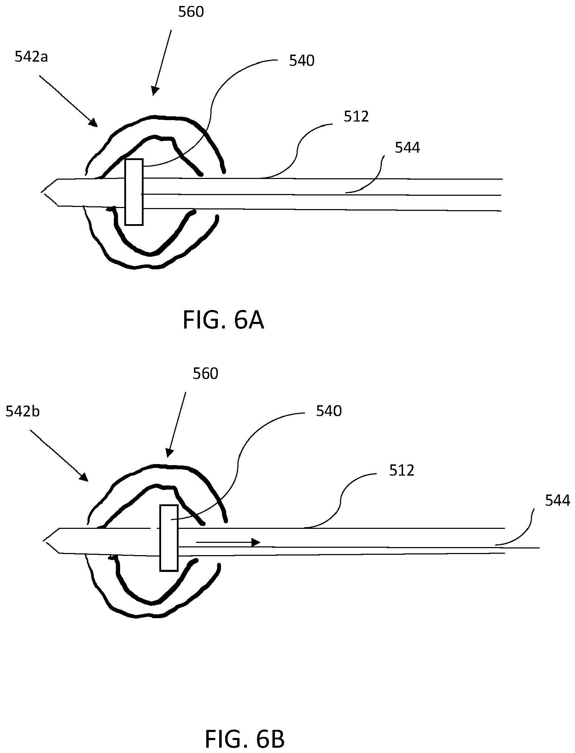

Figs. 6A and 6B illustrate an example transducer assembly including a transducer 540 that is configured to slide over an inner shaft 512 when the anchor is deployed. When the anchor 560is deployed, the transducer 540 is translated across the inner shaft 512 to ablate several ablation sites (e.g., a first ablation site 542a and a second ablation site 542b) at one anchoring position. The transducer 540 may be translated to one, two, three, four, five, or more ablation sites, or more if desired, at one anchoring position. This method of translating the transducer 540 can reduce treatment time by reducing the amount of times the anchor 500 is collapsed and moved, and then redeployed within a vessel. In some embodiments, the anchor 500 may be only partially collapsed or not collapsed before movement (e.g., it may be worth possible vessel wall damage to reduce procedure time by moving an at least partially expanded anchor). The transducer 540 may be connected to a pull and/or push wire 544 to move the transducer 540 along the inner shaft 512.

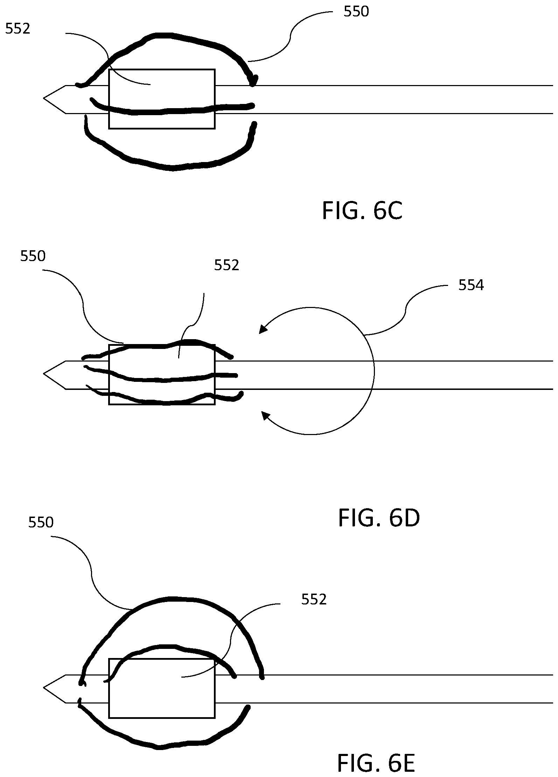

Figs. 6C-6E illustrate an example method of rotating an anchor 550 between ablations. Rotation of the anchor 550 may counteract or account for any interference caused by shadows created by the anchor 550. After a first ablation conducted in a deployed state as shown in Fig. 6C, the anchor 550 may be collapsed to a delivery state as shown in Fig. 6D. The anchor 550 may then be rotated as shown by the arrow 554. The anchor 550 is preferably not longitudinally moved during rotation. The anchor 550 is then redeployed with the struts touching a different portion of the vessel wall, as shown in Fig. 6E. The struts of the anchor 550 are in different positions than in Fig. 6C, which results in any interference or shadows occurring in different areas of the vessel, which can allow the transducer 552 to ablate the tissue where the shadows were cast in Fig. 6C, thereby providing a more complete ablation. This process will be repeated as many times as desired to account for interference or shadows.

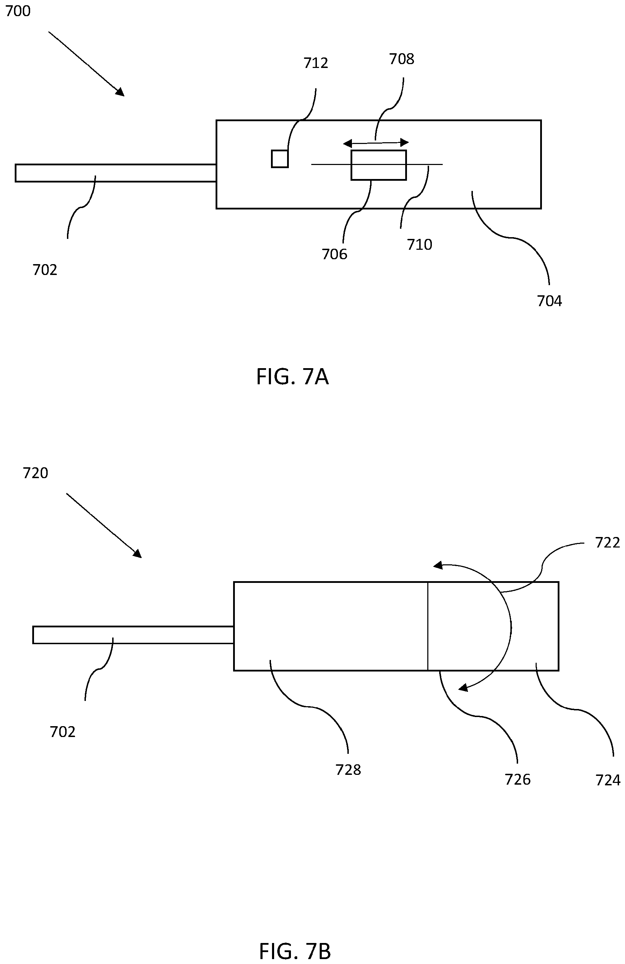

Fig. 7A schematically illustrates an example embodiment of a catheter 700 comprising a handle 704 and an elongate shaft 702. A distal portion of the elongate shaft 702 may comprise the transducer assembly, anchor, etc. The handle comprises an actuator 706. The actuator 706 may be used to collapse and/or deploy the anchor 500. The actuator 706 may comprise, for example, a thumb wheel or slider. In some embodiments in which the actuator 706 comprises a slider, the actuator 706 can slide along a path 710 in either direction, as indicated by the arrow 708. In some embodiments, the actuator 706 can inform the operator about the inner diameter of a vessel. For example, as described herein, the longitudinal distance between the distal shaft 502 and the proximal shaft 512 is related to the radial expansion of the struts 504. If the actuator 706 proximally retracts the distal shaft 502 by a certain distance, then the corresponding extent of the radial expansion of the struts 504, and thus the diameter of the vessel that stopped expansion of the struts 504, can be determined. The handle 704 may comprise indicia along the path 710. Vessel diameter information may be used to select the energy value (e.g., time and/or modulation) to increase the safety and the efficacy of the treatment.

In some embodiments, the handle 704 may comprise a button 712 configured to start ablation. A foot switch, a software button located on an instrument touch screen, a mouse click, and/or other ablation inciting inputs are possible.

In embodiments comprising an outer sheath, the handle 704 may comprise a mechanism to proximally retract and/or distally advance the outer sheath (e.g., a second actuator). In some embodiments, the outer sheath may be directly manipulated by the user (e.g., distal to the handle 704 and/or proximal to the handle 704).

In some embodiments, the handle 704 may comprise components for retracting the distal portion of the elongate shaft by a controlled distance between ablation sites, as described in additional detail herein. For example, the handle 704 may comprise a third actuator. If the handle 704 comprises a plurality of actuators, the actuators may be labeled with indicia (e.g., letters or numbers), comprise different colors, etc. Preferably, each of the actuators is at least partially different. For example, a plurality of actuators each configured to slide in a path may have different shapes, surface textures, colors, etc. In some embodiments, the actuators are distinguishable by being different types of actuators (e.g., thumb wheel for operation of the outer sheath, slider for deployment of the anchor, knob for controlled retraction of the distal portion, etc.).

Fig. 7B schematically illustrates another example embodiment of a catheter 720 comprising a handle 724 and an elongate shaft 702. The handle 724 may comprise the features of the handle 704. The handle 724 comprises a proximal part 726 and a distal part 728. The proximal part 726 may be rotated relative to the distal part 728 as shown by the arrow 722 to advance or retract the distal portion of the catheter 720 within a vessel. The rotation of the proximal part 726 is translated into linear motion advancing or retracting the distal portion of the catheter 720 within a vessel, for example using a helix, a worm gear, rack and pinion, etc. Rotating the proximal part 726 in one direction advances the distal portion of the catheter 720, and rotating the proximal part 726 in the opposite direction retracts the distal portion of the catheter 720. The handle 724 may comprise detents to help the user determine an appropriate amount of rotation and thus movement of the distal portion of the catheter 720. For example, each controlled turn of the proximal part 726 may proximally retract the distal portion of the catheter a set distance, for example between about 0.25 cm and about 2 cm (e.g., about 0.25 cm, about 0.5 cm, about 1 cm, about 1.5 cm, about 2 cm, and ranges between such values). Distal advancement of the distal portion is also possible.

In some embodiments, the distal portion of the catheter 720 is advanced to a first target location, such as a distal location in the LPA. The anchor is deployed and the tissue around the first target location is ablated. The anchor is then collapsed and the proximal part 724 is rotated to proximally retract the distal portion of the catheter 720, for example by 0.5 cm, to a second target location. The handle 724 may comprise an interlock that inhibits or prevents rotation of the proximal part 726 if the anchor is in a deployed state. The anchor is redeployed and the tissue around the second target location is ablated. This collapse, retract (or otherwise move), redeploy, ablate sequence can be repeated for the length of the LPA and then the length of the PT. The distal portion of the catheter 720 is then advanced to an nth target location, such as a distal location in the RPA (e.g., after user manipulation of a guidewire). The anchor is redeployed and the tissue around the nth target location is ablated. The collapse, retract (or otherwise move such as distally advance), redeploy, ablate sequence can be repeated for the length of the RPA.

Any sequence of treatment of pulmonary arteries is possible. For example: LPA, then PT, then RPA; RPA, then PT, then LPA; LPA, then RPA, then PT; RPA, then LPA, then PT; PT, then RPA, then LPA; PT, then LPA, then RPA. Preferably, the PT is ablated after the LPA or the RPA to reduce navigation. In some embodiments, the PT may be ablated after the LPA and after the RPA.

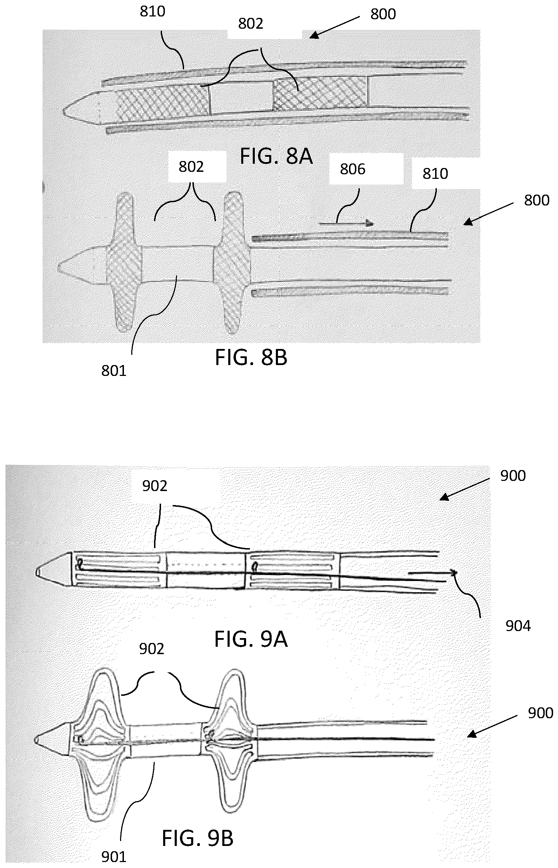

Figs. 8A and 8B illustrate another example embodiment of an anchor 800. Fig. 8A illustrates the anchor 800 in a collapsed state. In some embodiments, an outer sheath 810 inhibits or prevents the anchor 800 from expanding while in the collapsed state. Fig. 8B illustrates the anchor 800 in a deployed state. The anchor 800 comprises components on each side of the transducer assembly 801 801. The anchor 800 comprises two braid configurations 802. Additional braid configurations 802 are also possible. The first braid configuration 802 is distal to the transducer assembly 801, and the second braid configuration 802 is proximal to the transducer assembly 801. Braid configurations 802, for example having a high braid angle, can provide superior radial force compared to a plurality of struts having similar thicknesses, etc.

Figs. 9A and 9B illustrate another embodiment of an anchor 900. Fig. 9A illustrates the anchor 900 in a collapsed state. In some embodiments, an outer sheath 810 inhibits or prevents the anchor 800 from expanding while in the collapsed state. Fig. 9B illustrates the anchor 900 in a deployed state. The anchor 900 comprises a plurality of struts 902 on each side of the transducer assembly 901. The anchor 900 comprises two pluralities of struts 902. Additional pluralities of struts 902 are also possible. The plurality of struts 902 may be configured and operate in any of the ways the plurality of struts 504 are described herein. For example, the size and shape of the struts 902 may be any of the embodiments described herein, and the deploying and collapsing of the anchor 900 may occur in any of the ways described herein. Pluralities of struts 902 can provide simpler and/or more repeatable manufacturing compared to braid configurations 802, for example in terms of coupling to proximal and/or distal shafts.

In some embodiments, the anchor 800, 900 is deployed by pushing the braid configurations 802 or the pluralities of struts 902 together, causing the braid configurations 802 or the pluralities of struts 902 to bow radially outwards, as shown in Figs. 8B and 9B. In some embodiments, the anchor 800, 900 is self-expanding. The anchor 800, 900 may be collapsed and deployed by moving an outer sheath 810 (Figs. 8A and 8B) to cover or expose the anchor 800, 900. The outer sheath 810 is moved in the direction of the arrow 806 to deploy the anchor 800, 900. Additionally or alternatively, the anchor 800, 900 may be collapsed and deployed via a pull wire connected to one, some or all of the braid configurations 802 or the pluralities of struts 902. The anchor 800, 900 deploys when the pull wire(s) are pulled, and the anchor 800, 900 collapses when the pull wire(s) are advanced. In addition to or alternative to a pull wire, a shaft or tube could be used to push and/or pull a proximal and/or distal part of an anchor to deploy and/or collapse the anchor. In some embodiments, the pull wire(s) may be biased towards the collapsed state for a fail-collapsed configuration. In some embodiments, the fail-collapsed configuration could be achieved by heat shaping the anchor in the collapsed configuration. In certain such embodiments, a push mechanism could be used to achieve the deployed configuration. The anchor may be actuated and/or kept actuated by a wheel locker in a handle. Being fail-collapse can collapse the anchor upon failure (e.g., of the wire, shaft, etc.) while deployed in the subject.

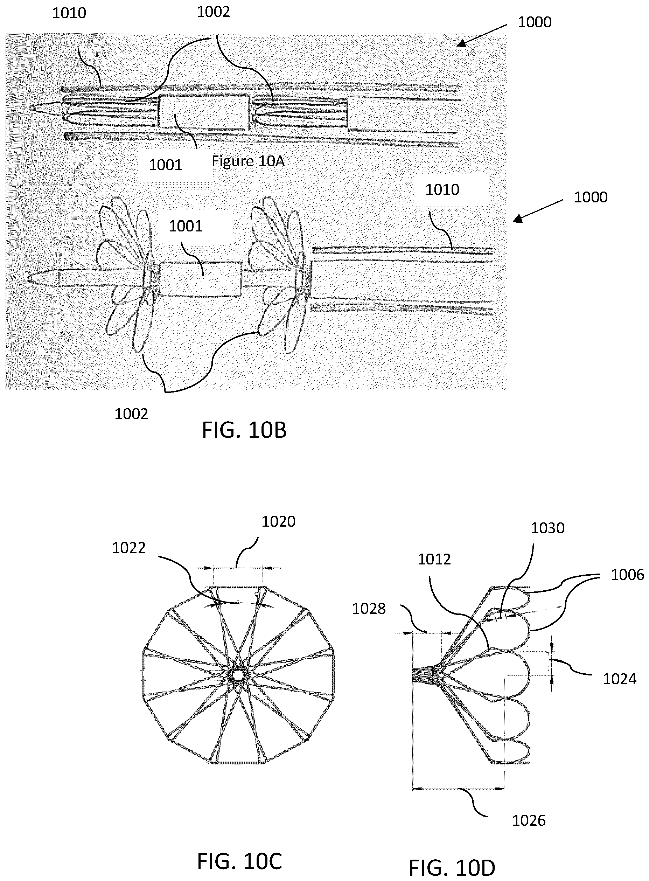

Figs. 10A-10D illustrate another embodiment of an anchor 1000. Fig. 10A illustrates the anchor 1000 in a collapsed state. In the embodiment illustrated in Fig. 10A, the outer sheath 1010 is inhibiting or preventing the anchor 1000 from radially expanding, for example causing stress-induced martensite. Fig. 10B illustrates the anchor 1000 in a deployed state. When the petal configurations 1002 are not confined by the outer sheath 1010, the anchor 1000 can self-expand due to a phase change to austenite. The anchor 1000 comprises a petal configuration 1002 on each side of the transducer assembly 1001. The anchor 1000 comprises two petal configurations 1002. Additional petal configurations 1002 are also possible.

The petal configurations 1002 comprise one or more wires shaped as a flower with multiple petals 1006. The petals 1006 may circumferentially overlap. The wire(s) may be shape set in the deployed state so that the petal configurations 1002 are self-expanding. In some embodiments, the anchor 1000 includes a float section (e.g., a segment generally parallel to the longitudinal axis) at the tip of the petals to increase the contact surface between the anchor 1000 and the vessel wall. The increase in contact surface may reduce the radial force applied to the vessel wall while still achieving the same anchoring (e.g., providing a substantially constant transducer assembly 1001 position under the same forces such as blood flow).

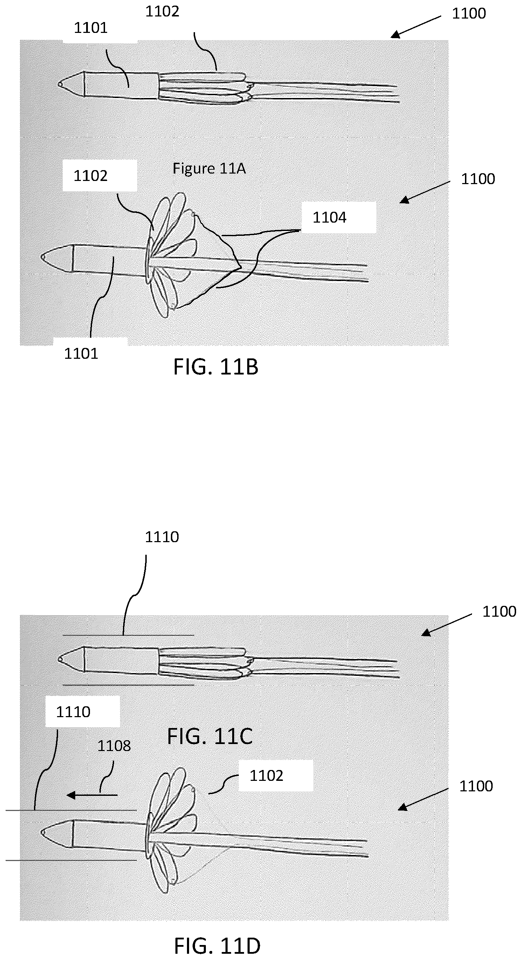

The anchor 1000 may be configured in multiple orientations. The petal configurations 1002 may be oriented to open facing the distal portion of the catheter, for example as shown in Fig. 10B. The petal configurations 1002 may be configured to face the handle (e.g., as described herein) of the catheter, for example as shown in Figs. 11A-11D. The petal configurations 1002 may be configured to face each other. The petal configurations 1002 may be configured to face away from each other.

The anchor 1000 may be self-expanding. The anchor 1000 may be collapsed and deployed by moving an outer sheath 1010 to cover or expose the petal configurations 1002. In some embodiments, the anchor 1000 is collapsed and deployed via a pull wire. If a petal configuration 1002 faces the handle, a pull wire may be used to collapse the petal configuration 1002 that is not collapsible by an outer sheath 1010 due to the direction the petal configuration 1002 is facing.

Fig. 10C is a top view of an example petal configuration for the anchor 1000 of Fig. 10A. The petals may have a circumferential width 1020 between about 5 mm and about 15 mm (e.g., about 5 mm, about 7 mm, about 9 mm, about 11 mm, about 13 mm, about 15 mm, and ranges between such values). The base of the petal configurations may be configured to create angles 1022 of between about 10 degrees and about 20 degrees (e.g., about 10 degrees, about 12 degrees, about 14 degrees, about 16 degrees, about 18 degrees, about 20 degrees, and ranges between such values).

Fig. 10D is a side view of the example petal configuration of Fig. 10A. The top portion of the petals may have a radius 1024 between about 1 mm and about 8 mm (e.g., about 1 mm, about 2 mm, about 3 mm, about 4 mm, about 5 mm, about 6 mm, about 7 mm, about 8 mm, and ranges between such values). The distance 1026 between the base of the petal configuration and the center of the diameter of the petals may be between about 12 mm and about 20 mm (e.g., about 12 mm, about 14 mm, about 16 mm, about 18 mm, about 20 mm, and ranges between such values). The distance 1028 between the base of the petal configuration and start of the petals may be between about 2 mm and about 8 mm (e.g., about 2 mm, about 3 mm, about 4 mm, about 5 mm, about 6 mm, about 7 mm, about 8 mm, and ranges between such values). The distance 1030 between the angle portion of the petal and the start of the arc of the petal may be between about 0.5 mm and about 2.5 mm (e.g., about 0.5 mm, about 1 mm, about 1.5 mm, about 2 mm, about 2.5 mm, and ranges between such values).

The anchors 800, 900, 1000 can apply a radial force on the vessel wall to anchor the transducer assembly 801, 901, 1001 within the vessel. The anchor 800, 900, 1000 is configured to conform to the different diameters of the vessels, as described herein. For example, the PT is typically larger in diameter than the LPA and RPA and the anchor 800, 900, 1000 expands according to the diameter of the ablation site. Depending on the diameter of the vessel, it may not be possible to achieve the delivery states shown in Figs. 8B, 9B, and 10B, but expansion of the anchor 800, 900, 1000 such that the anchor 800, 900, 1000 is able to maintain a substantially constant position of the transducer assembly 801, 901, 1001 in the vessel may be considered the deployed state. Ablation preferably occurs when the anchor 800, 900, 1000 is in the deployed state, or is not in the delivery state.

The anchors 800, 900, 1000 are proximal and distal to the transducer assemblies 801, 901, 1001, respectively. The anchors 800, 900, 1000 do not longitudinally overlap with the transducer assemblies 801, 901, 1001 and do not cast shadows, scatter acoustic energy, or otherwise block ablation energy. The anchors 800, 900, 1000 can allow a single ablation without rotation because the ablation energy can be circumferential and not blocked.

Figs. 8A and 8B illustrate two braid configurations 802, Figs. 9A and 9B illustrate two pluralities of struts 902, and Figs. 10A-10D illustrate two petal configurations 1002, but some embodiments of anchors may comprise any number of braid configurations, pluralities of struts, petal configurations, combinations thereof, and/or the like. For example, an anchor may comprise one braid configuration and one plurality of struts, one braid configuration and one petal configuration, or one plurality of struts and one petal configuration, for example to provide certain benefits of each type of anchor.

Figs. 11A-11D illustrate another embodiment of an anchor 1100. Fig. 11A illustrates the anchor 1100 in a collapsed state. Fig. 11B illustrates the anchor 1100 in a deployed state. The anchor 100 comprises one petal configuration 1102. The petal configuration 1102 may be configured in any of the described embodiments of petal configurations 1002.

The anchor 1100 can be configured with the petal configuration 1102 facing proximally. When the petal configuration 1102 is proximal to the transducer assembly 1101, the petal configuration 1102 faces away from the transducer assembly 1101 (e.g., as shown in Fig. 11B). When the petal configuration 1102 is distal to the transducer assembly 1101, the petal configuration 1102 faces towards the transducer assembly 1101. The anchor 1100 may be deployed and collapsed via an outer sheath 1110 and/or pull wire(s) 1104 connected to one, some, or all of the petals of the petal configuration 1102. If the pull wire(s) 1104 is not connected to all petals, the overlapping of the petals can cause all petals to be collapsed when the pull wire 1104 is pulled. Figs. 11C and 11D illustrate the use of an outer sheath 1110 to deploy and collapse the anchor 1100. The outer sheath 1110 is positioned on the distal portion of the catheter. The outer sheath 1110 is distally advanced in the direction of the arrow 1108 to allow the anchor 1100 to expand to the deployed state. The outer sheath 1110 is then proximally retracted to collapse the anchor 1100.

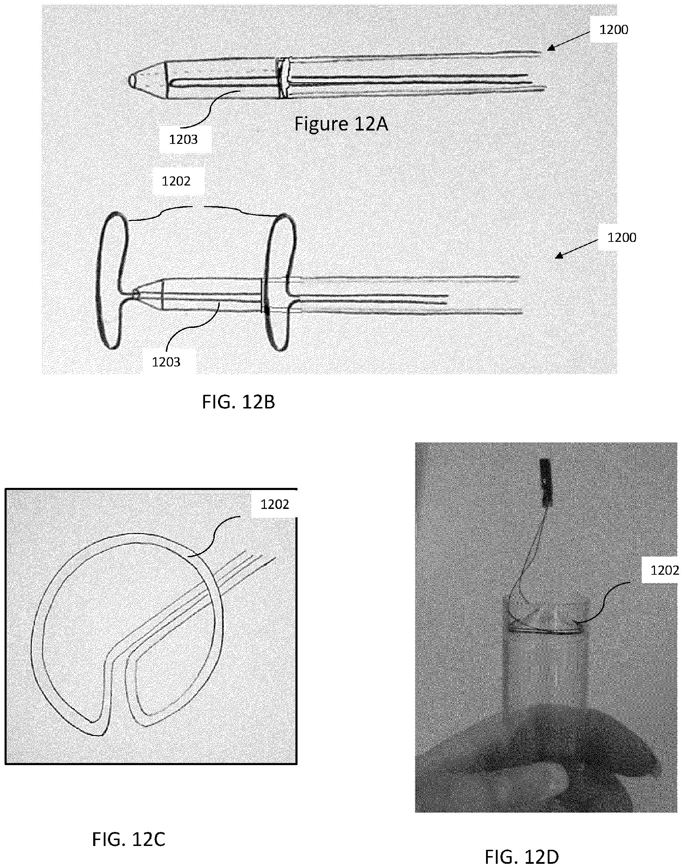

Figs. 12A-12D illustrate another embodiment of an anchor 1200. The anchor 1200 comprises a loop wire 1202. The anchor 1200 may comprise one, two, or more loop wires 1202. Figs. 12C and 12D illustrate the loop wire 1202. Fig. 12A illustrates the anchor 1200 in a collapsed state. An outer sheath as described herein may be used to inhibit or prevent the anchor 1200 from expanding. Fig. 12B illustrates the anchor 1200 in a deployed state.

The loop wires 1202 may be positioned distal and proximal of transducer assembly 1203 anchor the transducer assembly 1203 in a vessel. In embodiments comprising a single loop wire 1202, the loop wire 1202 may be located distal to or proximal to the transducer assembly 1203. In some embodiments, the loop wire 1202 is self-expanding and can be actuated by pushing the wire (e.g., one or both legs) from the proximal side of the catheter. The loop wire 1202 is then collapsed by pulling the wire.

All embodiments of the anchor described herein may be modified and combined to create additional embodiments. For example, all embodiments may consist of one, two, three or four anchors. In embodiments comprising more than one anchor, the anchors may be of different types. For example, one embodiment of an anchor may comprise a plurality of struts and a braid configuration. Any combination of the disclosed embodiments may be possible. All methods of deploying and collapsing the different anchor embodiments may apply to any of the anchor embodiments, including but not limited to, the umbrella method, the movement of an outer sheath, the use of a pull wire, the use of actuating shafts (e.g., telescoping shafts), and the use of self-expanding material. In embodiments in which neuromodulation is provided by, for example, acoustic energy (e.g., ultrasound), microwave energy, radiofrequency (RF) energy, thermal energy, electrical energy, infrared energy, laser energy, phototherapy, plasma energy, ionizing energy, mechanical energy, cryoablation, chemical energy, combinations thereof, and the like, the anchor may optionally push the transducer or other element against the vessel wall.

### Suspension

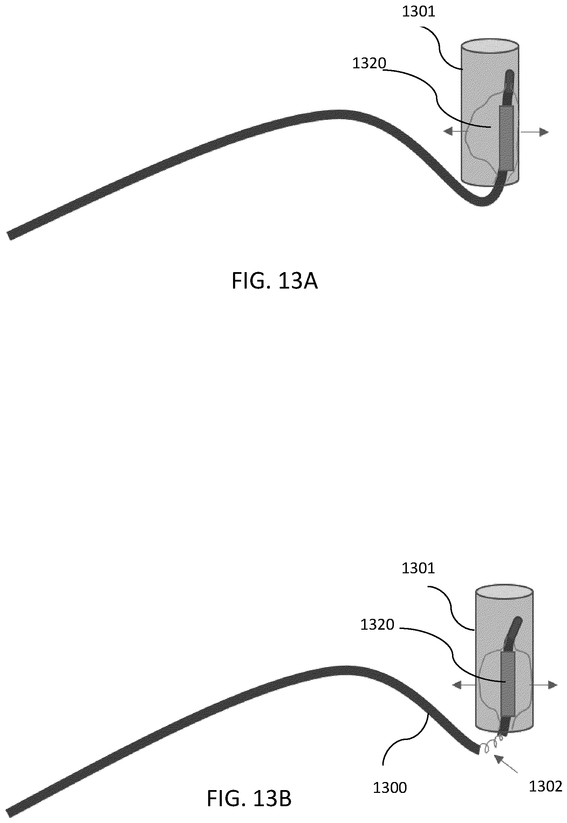

The distal portion of the catheter (e.g., the catheter 10) is flexible enough to navigates a variety of vessels, and cavities such as heart chambers, and rigid enough to be advanced through valves such as the tricuspid valve and the pulmonary valve. This combination of flexibility and rigidity may cause undesirable effects when the distal portion is anchored ablation. Fig. 13A illustrates an example catheter in a vessel 1301 that is not properly anchored. As shown in Fig. 13A, the transducer assembly 1320 is supposed to be anchored for ablation. The curvature of the catheter proximate to the anchor pushes the transducer assembly 1320 to the right side because the radial force of the anchor is not able to overcome the force of the catheter.

Fig. 13B illustrates an example embodiment of a catheter in which the stiffness of the shaft 1300 can be effectively negated proximate the distal portion. To reduce the impact of the shaft 1300 stiffness, some embodiments comprise a suspension 1302. The suspension 1302 may comprise a coil or other type of flexible shaft portion configured to release some of the constraints due to the shaft 1300 stiffness and curvature proximate the distal portion. The suspension 1302 is more flexible then the shaft 1300, which can allow the distal portion to effectively ignore the forces of the shaft 1300, which are absorbed by the suspension 1302. The suspension 1302 can provide better anchoring and centering of the transducer assembly 1320. The suspension 1320 may comprise any suitably flexible material.

### Movement Features

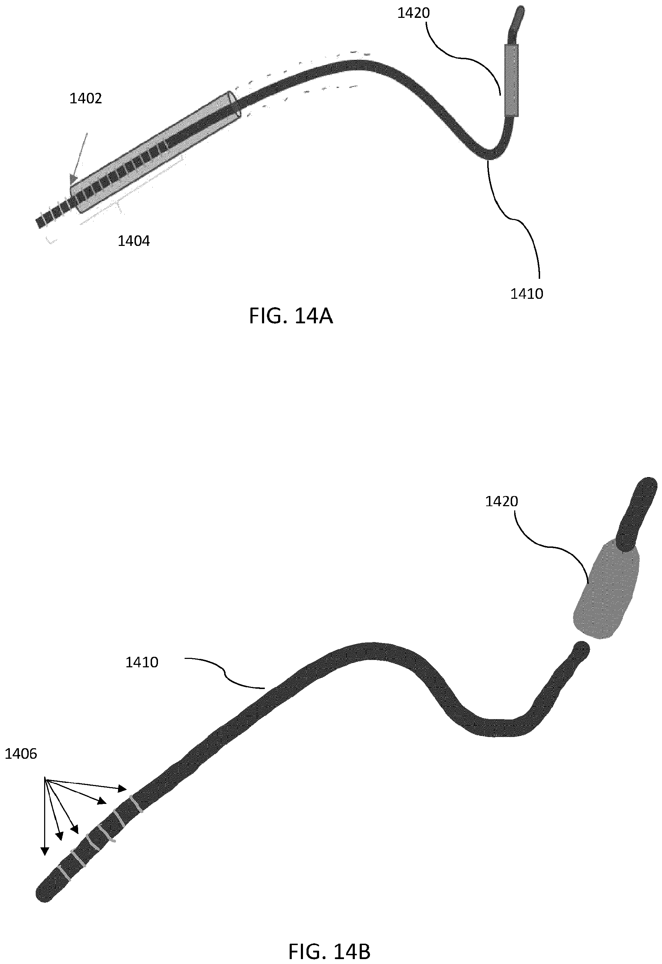

The distal portion of the catheter (e.g., the catheter 10) is navigated through vessels to multiple ablation sites. The distance between ablation sites may be controlled (e.g., as described with respect to the handle 724) and/or monitored. The movement (e.g., retraction, advancement) features described herein may be used to monitor the distance between ablation sites. Fig. 14A illustrates a distal portion of a catheter comprising a shaft 1410 and a transducer assembly 1420. The catheter is configured to enter the patient at a vein access point 1402. Vein access points include but are not limited to femoral, jugular, and radial access points. Any suitable vein access point may be used.

The shaft 1410 may comprise electrodes 1404 located along a proximal portion of the shaft 1410. The electrodes 1404 are configured to sense the electrical conduction between each electrode to determine the distance the transducer assembly 1420 was pulled or pushed from an ablation site. In some embodiments, the conduction between a first set of electrodes are high impedance, while the conduction between the rest of the electrodes is low impedance. A variance between low and high impedance may be used to account for the electrical conductivity of the blood that is in contact with the electrodes positioned within the body. For example, the electrodes 1404 outside the vein access point 1402 in Fig. 14A will have a high impedance, while the electrodes 1404 within the vein will have a lower impedance.

In some embodiments, the electrodes 1404 are located at fixed points along the shaft 1410. The fixed locations allow software running on an instrument (e.g., as described herein) to detect the number of electrodes 1404 moved in or out of the body. Tracking the movement of electrodes 1404 may be used to determine the approximate distance between positions of the transducer assembly 1420 and the different ablation sites. In some embodiments, data about the transducer assembly position, diameter of the deployed anchor, and/or ablation parameters can be stored. A report can be produced. Reports from the treatment of various subjects can be combined with data about the effectiveness of the treatment for those subjects to improve the system (e.g., determining ideal ablation spacing, ablation parameters, etc.). Embodiments comprising electronics may comprise interlocks, for example inhibiting or preventing an ablation until the catheter has been moved to a different ablation site.

Fig. 14B illustrates another example embodiment of movement (e.g., retraction, advancement) features. The shaft 1410 comprises marks or indicia 1406. Any number of marks 1406 along the shaft 1410 can be used. The marks 1406 can be separated any distance, for example every half centimeter. The marks 1406 allow the operator to control and monitor the distance between two ablation sites when pulling or pushing the catheter. For example, the marks 1406 may be compared to a stationary object (e.g., an access point). Some embodiments may include additional and/or alternative methods to control the distance between two ablation sites when pulling or pushing the catheter. For example, an actuator (e.g., the actuator 706) may be configured to push or pull the catheter a specified distance with each actuation. For another example, magnetic beacons can be used. For another example, a wheel with appropriate gearing can be used.

In some embodiments, the movement (e.g., retraction, advancement) feature may comprise radiopaque markers on the distal portion of the catheter that can be observed under fluoroscopy. Such a movement feature may provide the ability to make sure that the movement of the catheter (e.g., by manipulating a handle) translates into the expected or desired movement in the vessel. Fluoroscopy can also or alternatively be used in combination with any of the movement features described herein.

### Ablation Instruments

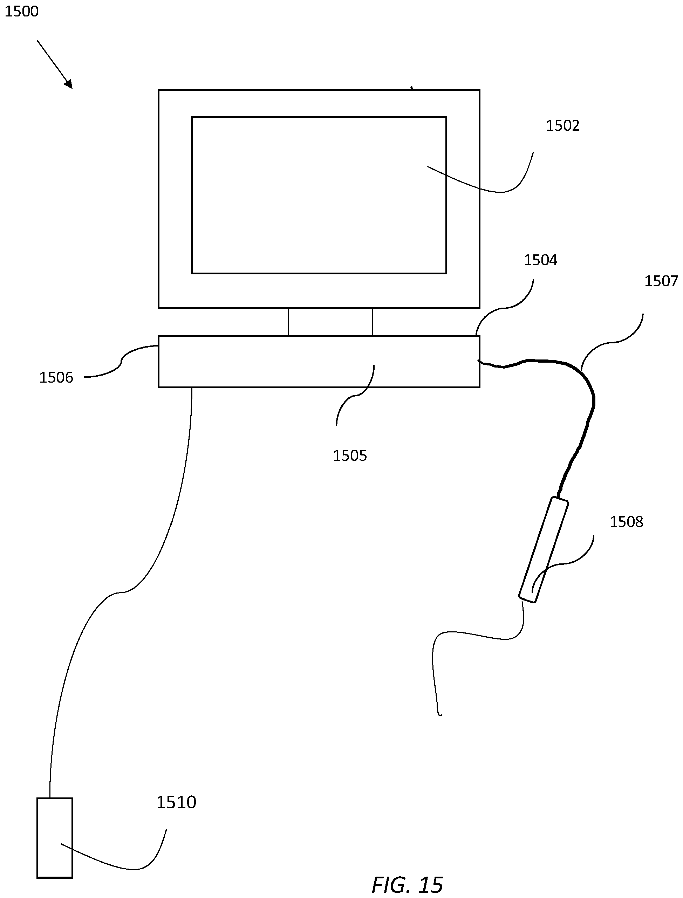

Fig. 15 is a schematic diagram of an example ablation instrument 1500. The instrument 1500 serves as the user interface and provides the electrical power to a catheter 1508. The instrument 1500 includes a display screen 1502, an ultrasound beam generator 1504, a power monitor 1505, a control computer 1506, a removable catheter connector 1507 between the control computer 1506 and a catheter 1508, and a foot pedal 1510 that may be used to initiate ablation. The display screen 1502 may be a touch screen. The instrument 1500 may comprise other inputs (e.g., a mouse, a keyboard, a track ball, etc.).

### Ultrasound Beam Generator

The ultrasound beam generator 1504 comprises an electrical power amplifier with an output between 1.5 MHz to 11 MHz capable of 200 Watts or more of electrical power in continuous wave mode or in pulse wave mode. The ultrasound beam generator 1504 supports a programmatic interface, for example through an internal USB to Serial port interface. The interface allows the control computer 1506 to start or stop the ultrasound emission. The ultrasound beam generator 1504 can embed a firmware in charge of the pulse emission communication with the control computer 1504 to check internal devices such as temperature sensors (e.g., as described herein), fans, etc.

The tissue around the pulmonary artery, which may include nerves, can be ablated by applying ultrasound energy to the transducer, which is focused by the lens. The energy can be applied for a duration between about 0.5 seconds and about 1 minute (e.g., about 0.5 seconds, about 1 second, about 2 seconds, about 3 seconds, about 4 seconds, about 5 seconds, about 6 seconds, about 7 seconds, about 8 seconds, about 9 seconds, about 10 seconds, about 15 seconds, about 30 seconds, about 45 seconds, about 1 minute, and ranges between such values).

The energy can be between about 20 Watts (W) and about 80 W acoustic (e.g., about 20 W, about 30 W, about 40 W, about 50 W, about 60 W, about 70 W, about 80 W, and ranges between such values). The acoustic wattage is at least partially based on electric power applied and the efficiency of the system such as the transducer assembly. For example, if the system is 50% efficient, the application of 40 W electric would be 20 W acoustic. If transducer assemblies are between about 50% and about 80% efficient, then the electrical power applied can be between about 25 W and about 160 W to produce between about 20 W and about 80 W acoustic.

Although described herein with respect to ultrasound, other energy modalities are also provided, for example unfocused ultrasound, focused ultrasound such as high-intensity or low-intensity focused ultrasound, microwave energy, radiofrequency (RF) energy (e.g., monopolar, bipolar, etc.), thermal energy (e.g., cryoenergy, heat or cold provided by a fluid (e.g., water, saline, liquid medicament, etc.) or gas (e.g., steam)), electrical energy (e.g., non-RF electrical energy), infrared energy, laser energy, phototherapy or photodynamic therapy (e.g., in combination with one or more activation agents), plasma energy, ionizing energy delivery (e.g., X-ray, proton beam, gamma rays, electron beams, alpha rays, etc.), mechanical energies delivered by cutting or abrasive elements, cryoablation, chemical energy or modulation (e.g., chemoablation), or combinations thereof. In some embodiments, disruption or interruption of nerves is carried out by chemicals or therapeutic agents (for example, via drug delivery), either alone or in combination with an energy modality. In some embodiments, pharmaceuticals are combined with the neuromodulation (e.g., ablation) described herein to reduce the dosage or duration of pharmacology therapy, thus reducing side effects. In various embodiments, different energy modalities may be used in combination (either simultaneously or sequentially).

### Power Monitor

The power monitor 1505 measures the electrical power using a directional coupler. The directional coupler comprises two coils with ferrite to measure power without inducing a loss due to measurement. The power monitor 1505 measures the power being sent to the transducer (forward power) and the power being reflected back (reverse power). The forward or the reverse power are measured through an Analog to Digital Converter that are read in real-time by the control computer through an internal USB interface.

The efficiency and natural frequency of each catheter, transducer, and/or transducer assembly may be measured prior to use, for example by the manufacturer, another facility, an independent company, and/or the like.

During the ablation procedure, the user inputs the efficiency and the natural frequency of the transducer being used. Each system can include an indicator of the efficiency of that particular system so that the ultrasound beam generator can account for losses to deliver the appropriate acoustic energy. The indicator may be a fact sheet that is input by a user. The fact sheet may be a sticker on the box, on the instructions for use, on a sterile wrapper, on a package insert, and/or the like. The indicator may be a bar code or QR code that may be read by an appropriate device. The indicator may be embedded in a flash memory such as an EPROM that can be automatically read by the ultrasound beam generator when the catheter 1508 is coupled to the connector 1507. The memory may be in a USB stick, a SD card, or other hard media that may be required to be inserted in the control computer 1506 for the system to function. The beam generator can use information from the indicator to ensure that a catheter is not reused for multiple procedures (e.g., at all, unless a user indicates appropriate sterilization, etc.). A simpler indicator may reduce costs. A more complicated indicator can reduce the risk of user error.

During use, the power monitor 1505 will monitor the reverse power (unused power that is reflected back) and compare it to the expected results from the inputted data. If the reverse power losses are calculated as being too high or indicate a broken transducer (or any problem with the transducer), the procedure can be stopped. For example, if there is too much reverse power, the energy is not converted into acoustic and therefore the system is in some variety of failure (e.g., broken cable linking generator and transducer, solder failure, too many bubbles reflecting the power back to the source, parasitic capacitance, etc.).

### Control Computer

The control computer 1506 is configured to assist the user during a procedure. The control computer 1506 controls the user interface, drives the power generator, and controls the power output. For example, the control computer 1506 may be loaded with data from a planning tool to assist in ablation. This data may comprise ablation site positions, diameters of the vessels, distances between ablation sites, etc. The pre-loaded data may comprise data that was previously collected via CT scan images, MRIs, IVUS, or other medical scans, images, tests of the patient, etc. By knowing this information prior to the procedure, the user may define the diameter of the artery at the ablation site using the control computer 1506 to set or optimize the acoustic power and the pulse duration. After the initial phase of positioning the catheter, the treatment may then be automatically monitored using the electrodes, described herein, to generate a treatment report.

The treatment report may include a report of the power delivered at each ablation site. A report of the power delivered will increase the user's overall efficiency and capability from procedure to procedure. The report may also indicate the different sizes of toroidal ablation based on vessel size. For example, the smaller the vessel, the smaller the toroidal ablation site should be. If the reported size varies from the expected size, the user may adapt the power or time of ablation based on the vessel size. In some embodiments, the anchor may be configured to measure the vessel size to be included in the treatment report.

### Sensors

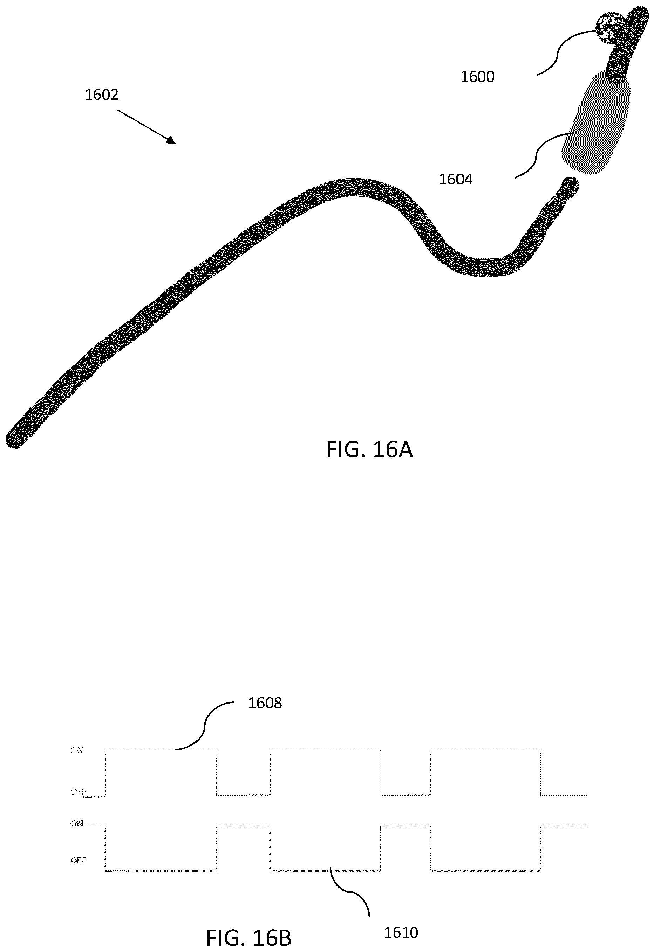

Sensors (e.g., sensor 1600) may be used to monitor different values during ablation. Fig. 16A illustrates an example catheter 1602 comprising a sensor 1600 located on a distal portion of a catheter 1602. The sensor 1600 may be positioned distal to the transducer 1604, as shown, proximal to the transducer 1604, or in any other suitable configuration. The sensor 1600 may be configured to monitor temperature to track safety and efficiency of the ablation procedure.

Fig. 16B is a graph depicting temperature measurement 1610 and pulse emission 1608 during ablation. As shown in Fig. 16B, the temperature measurement 1610 should be consistent while ablating. The pulse emission 1608 should also be consistent during ablation. The sensor may be configured to indicate if there is a change in temperature or an unexpected temperature. For example, a temperature that is too high may indicate that there is something wrong and that the procedure should be stopped. The temperature measurement samples in between two pulse emissions 1608 may work around the viscous heating effect of the thermocouple measurement while the thermocouple is located inside the ultrasound beam. This viscous heating effect can be an artifact that rises the temperature value and could lead to wrong measurement. The sensor 1600 may also be configured to measure other values such as blood pressure, flow rate, heart rate, and/or any measurement that may be relevant to the procedure or safety of the patient. Any measurements taken, such as blood pressure, may be used to synchronize the ultrasound emission with the measurement taken.

In some embodiments, the transducer assembly could be used to measure the efficiency by measuring a returned signal during the neuromodulation. For example, during a pulse emission, some energy is reflected back to the transducer when the ultrasound wave travels through an interface between media. When tissue heats, the characteristic of that medium changes, and the change in the energy reflected back from an interface including that medium can be detected using the transducer as a sensor. The reflected energy may change the impedance of the transducer assembly, which can induce a modification of the reflected power returned back to the generator. The reflected power signal analysis can be used to detect a threshold when the pulse starts to be efficient enough, for example, to ablate the tissue. This information could be used to stop the pulse emission when the heating is sufficient for the nerve denaturation. In some embodiments, a multielement ultrasound probe having a cylindrical shape could be added to the system, separate from the transducer used for the neuromodulation, to perform ultrasound thermometry from the inside of the lumen and inform on the procedure efficacy.

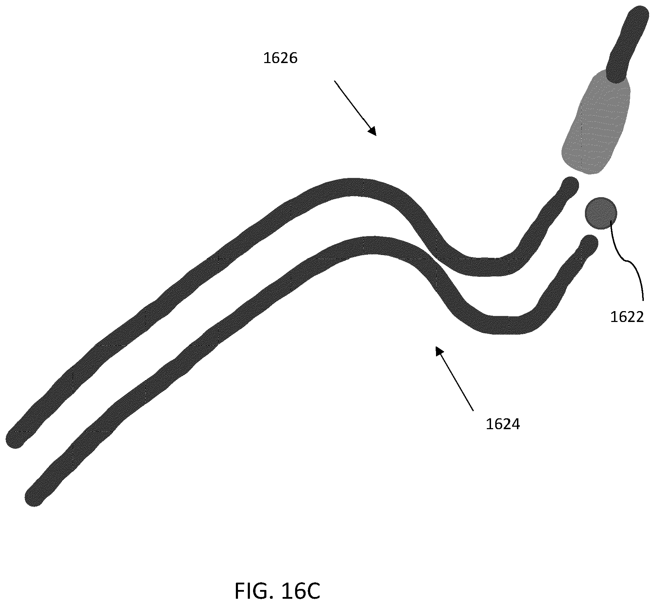

Fig. 16C illustrates an example catheter system including a second catheter 1624 embodiment comprising a sensor 1622. The second catheter 1624 is separate from the catheter 1626 comprising the transducer. The sensor 1622 being on a second catheter 1624 can increase the flexibility of where measurements may be taken. For example, the second catheter 1624 may be positioned in a different vessel than the first catheter 1626 or in a different location within the same vessel as the first catheter 1626. In some embodiments, the first catheter 1626 may comprise a lumen (e.g., having an exit port proximal to the transducer) to help guide the second catheter 1624 proximate to its intended position.

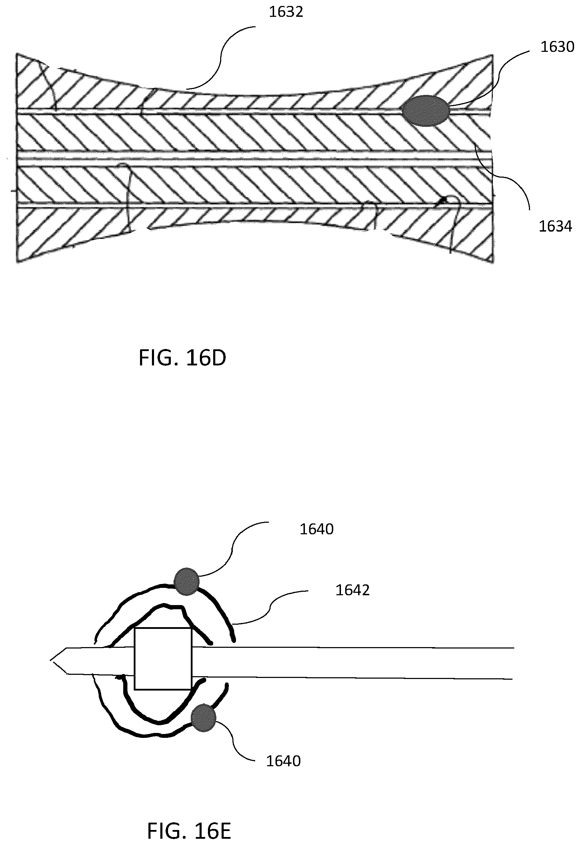

Fig. 16D illustrates a sensor 1630 coupled to the interior of a lens 1632. The sensor 1630 is configured to measure the lens temperature. The sensor 1630 may be a thermocouple sensor. This temperature may be monitored to inhibit or prevent overheating of the transducer 1634 to protect the transducer 1634 from being damaged. The temperature of the lens 1632 may be monitored because a lens that has too high a temperature may create clots in a patient.

Fig. 16E illustrates a plurality of sensors 1640 located on an anchor 1642. The sensors 1640 may be thermocouple sensors. One sensor 1640 may be used. In some embodiments, one, some, or all of the struts or other components (e.g., petals) comprises a sensor 1640. In some embodiments, some struts comprise a sensor 1640. The sensors 1640 may be used to measure the temperature next to a vessel wall.

### Methods

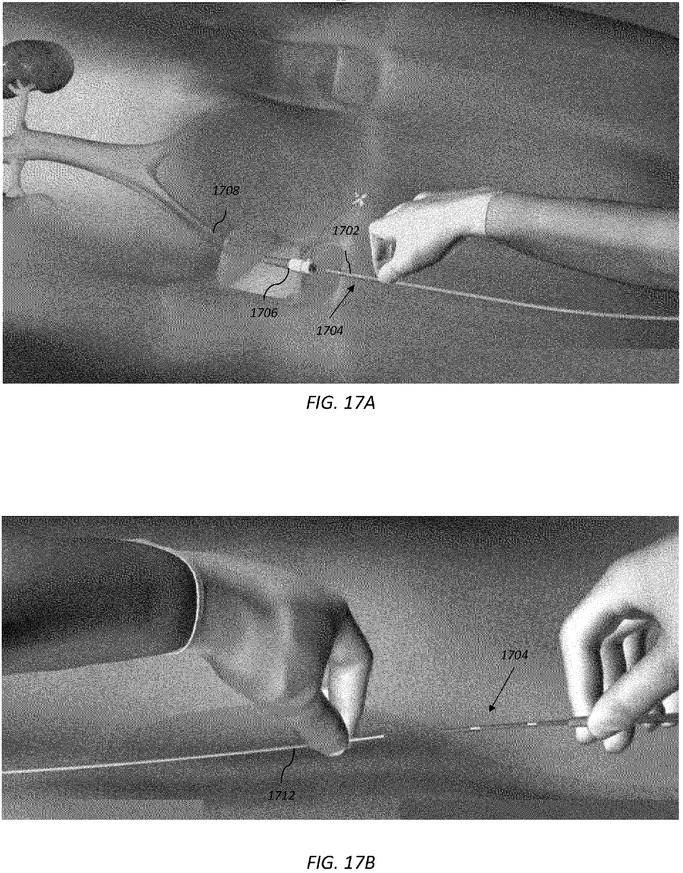

Ablation using any embodiment of the device described herein may occur at multiple ablation sites using a collapse and deploy method. Fig. 17A illustrates the positioning of a first catheter 1704 in a vein 1708 at an insertion site or vein access point 1706. The first catheter 1704 may comprise a balloon 1702. The first catheter 1704 may be positioned in the vein 1708, and the balloon 1702 may then be inflated. The inflated balloon 1702 may then be carried by blood in the venous vasculature, through the right heart, and to a first pulmonary artery. A guidewire 1712 may then be navigated to the first pulmonary artery and the first catheter 1704 may be removed. Fig. 17B illustrates a treatment catheter 1704 being positioned over the guidewire 1712. The treatment catheter 1704 is tracked over the guidewire 1712 to the first pulmonary artery. The treatment catheter 1704 may be any of the previously described embodiments.

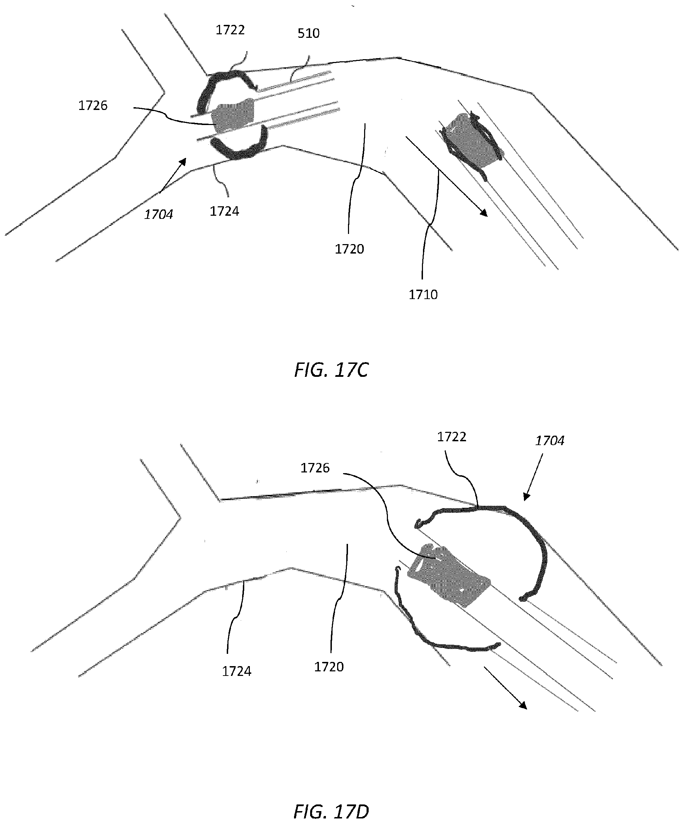

Fig. 17C illustrates the distal portion of the shaft of the treatment catheter 1704 being positioned within the right pulmonary artery (RPA) 1720. The anchor 1722 has been positioned and deployed, according to any of the methods described herein, within the RPA 1720 at a first ablation site. The anchor 1722 anchors the transducer 1726 within the RPA 1720. The anchor 1722 deploys to contact the artery wall 1724 applying a radial force. After deploying the anchor 1722, the tissue surrounding the first ablation site (e.g., including nerves) is ablated. Interrupting the nerves around the RPA 1720 can reduce pulmonary hypertension. In some embodiments, neuromodulation is accomplished (e.g., via ablation, denervation, which may or may not be reversible, stimulation, etc.). Ablation may occur at one location during the deployed state, or the transducer 1726 may be translated as described herein to perform multiple ablations during a single deployed anchor position.

An ablation site may be ablated for between about 0.5 seconds and about 1 minute (e.g., about 0.5 seconds, about 1 second, about 5 seconds, about 30 seconds, about 1 minute and ranges between such values). The frequency used during ablation may be between about 1.5 MHz and about 11 MHz (e.g., about 1.5 MHz, about 2 MHz, about 2.5 MHz, about 3.5 MHz, about 4.5 MHz, about 6 MHz, about 7.5 MHz, about 9 MHz, about 11 MHz, and ranges between such values). The acoustic power used during ablation may be between about 20 W and about 80 W (e.g., about 20 W, about 30 W, about 40 W, about 50 W, about 60 W, about 70 W, about 80 W, and ranges between such values). This translates to electric power of between about 25 W and about 160 W and ranges between such values.

Each ablation site may be of a different diameter. As shown in Figs. 17C-17I, not all diameters of the pulmonary arteries are the same. The anchor 1722 may be deployable to accommodate the different diameters, as described herein. The locations being ablated may be at different depths or focal points within the vessel walls. The ablation power and time or frequency of the ultrasound beam may be varied to accommodate the varying diameters and depths of the locations to be ablated. In some embodiments, a single set of ablation parameters (e.g., power, duration, frequency) could be used to accommodate various artery diameters. For example, the parameters (e.g., power, duration, frequency) could be set to a target range of lesion depth could be set to exclude tissue where ablation should not occur. In some embodiments, each ablation could include 50 W for one minute followed by 100 W for at least 30 seconds, with optional additional pulses at 100 W for particular locations.

After a first ablation site has been ablated, the anchor 1722 is collapsed by any of the methods described herein. The distal portion may then be retracted (or advanced) a distance within the RPA 1720, as shown by the arrow 1710 in Fig. 17C, and positioned and deployed at a second ablation site, as shown in Fig. 17D. The deploying, ablating, collapsing, and retracting steps may be repeated until the tissue around the desired amount of the RPA 1720 (e.g., the entire length of the RPA, 3/4, 2/3, 1/2, 1/3, 1/4, and ranges between such values) has been covered by the ablation. Because nerves can act like wires where cutting at any point along the length may be sufficient to disable the nerve, smaller lengths or segments of the RPA may be ablated to have a beneficial effect. Because nerves are not necessarily straight, can branch, can start or stop along the length of the RPA, etc. larger lengths may be used to have a beneficial effect. Ablation may be repeated at some or all ablation sites to account for any interference or shadows caused by the anchors, as discussed herein.

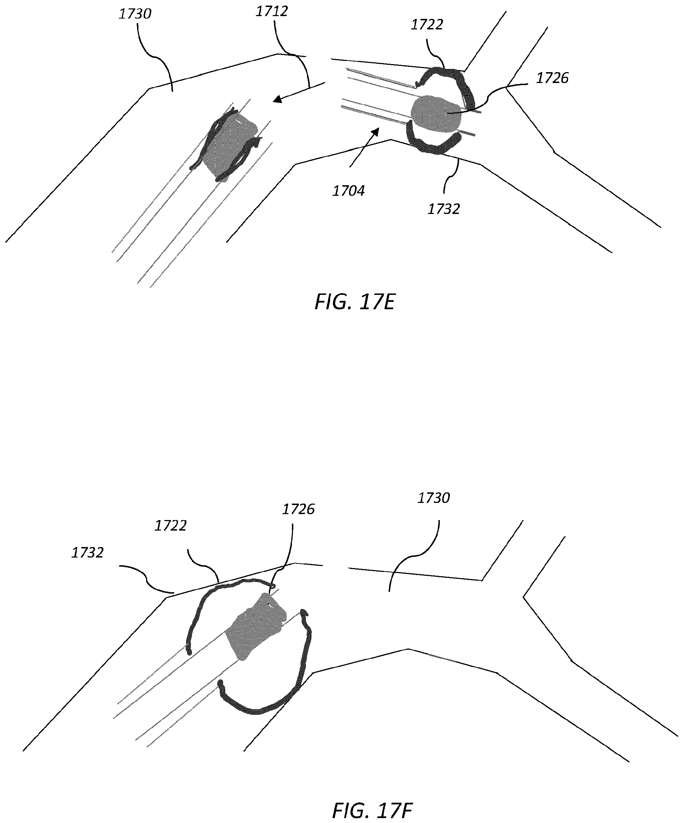

Fig. 17E illustrates the distal portion of the shaft of the treatment catheter 1704 positioned within the left pulmonary artery (LPA) 1730. The anchor 1722 is positioned and deployed, according to any of the above described methods, within the LPA 1730 at a first ablation site. The anchor 1722 anchors the transducer 1726 within the LPA 1730. The anchor 1722 deploys to contact the artery wall 1732 applying a radial force. After deploying the anchor 1722, the tissue surrounding the first ablation site (e.g., including nerves) is ablated. Interrupting the nerves around the LPA 1722 can reduce pulmonary hypertension. Ablation may occur at one location during the deployed state, or the transducer 1726 may be translated as described herein to perform multiple ablations during a single deployed anchor position.

Once the first ablation site has been ablated, the anchor 1722 may be collapsed by any of the methods described herein. The distal portion may then be retracted (or advanced) a distance within the LPA 1730, as shown by the arrow 1712 in Fig. 17E, and positioned and deployed at a second ablation site, as shown in Fig. 17F. The deploying, ablating, collapsing, and retracting steps may be repeated until the tissue around the desired amount of the LPA 1730 (e.g., the entire length of the LPA, 3/4, 2/3, 1/2, 1/3, 1/4, and ranges between such values) has been covered by the ablation. Because nerves can act like wires where cutting at any point along the length may be sufficient to disable the nerve, smaller lengths or segments of the LPA may be ablated to have a beneficial effect. Because nerves are not necessarily straight, can branch, can start or stop along the length of the LPA, etc. larger lengths may be used to have a beneficial effect. Ablation may be repeated at some or all ablation sites to account for any interference or shadows caused by the anchors, as discussed herein.

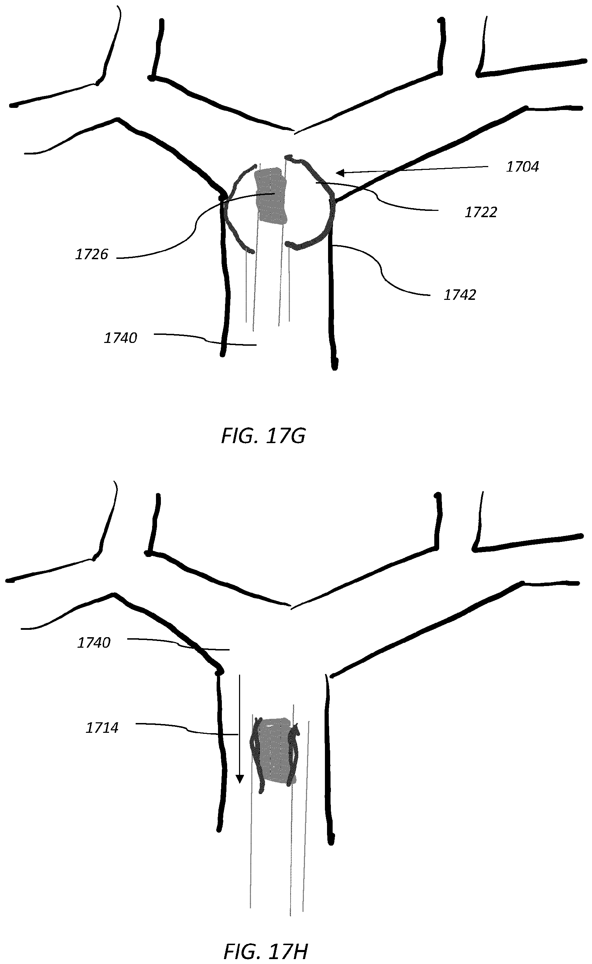

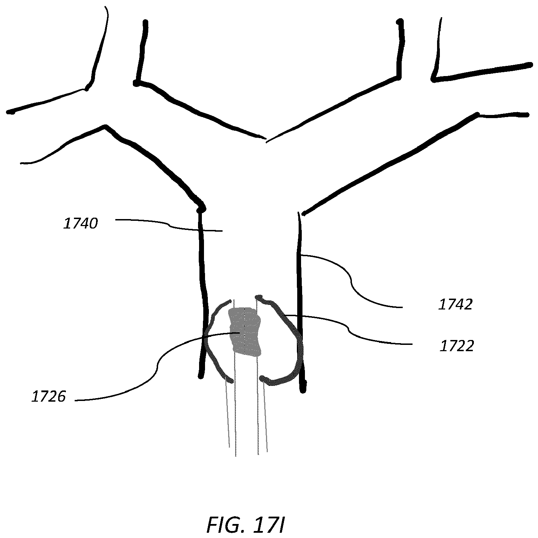

Fig. 17G illustrates a transducer 1726 positioned within a pulmonary trunk 1740 at a first ablation site. The anchor 1722 may be deployed by any of the described methods herein to anchor the transducer 1726 within the pulmonary trunk 1740. The anchor 1722 may be deployed to contact the pulmonary trunk walls 1742 applying a radial force to center the transducer 1726. The first ablation site may be ablated. Interrupting the nerves around the pulmonary trunk 1740 can reduce pulmonary hypertension. The anchor 1722 may be collapsed by any of the methods described herein. The transducer 1726 may then be retracted (or advanced) a distance within the pulmonary trunk 1720, as shown by the arrow 1714 in Fig. 17H, and positioned and deployed at a second ablation site, as shown in Fig. 17I. The second ablation site may be ablated. The deploying, ablating, collapsing, and retracting steps may be repeated until the tissue around the desired amount of the pulmonary trunk 1740 (e.g., the entire length pulmonary trunk, 3/4, 2/3, 1/2, 1/3, 1/4, and ranges between such values) has been covered by the ablation. Because nerves can act like wires where cutting at any point along the length may be sufficient to disable the nerve, smaller lengths or segments of the PT may be ablated to have a beneficial effect. Because nerves are not necessarily straight, can branch, can start or stop along the length of the PT, etc. larger lengths may be used to have a beneficial effect. Ablation may occur at one location during the deployed state, or the transducer 1726 may be translated as described herein to perform multiple ablations during a single deployed anchor position. Ablation may be repeated at some or all ablation sites to account for any interference or shadows caused by the anchors, as discussed herein. The treatment catheter may then be removed from the patient.

This method of ablation may be performed in any order. For example, as previously described, the right pulmonary artery (RPA) may be ablated first, followed by the left pulmonary artery (LPA), followed by the pulmonary trunk. Alternatively, the LPA may be ablated first, followed by the RPA, and followed by the pulmonary trunk. Any possible order may be used. If needed, but not necessary, ablation sites may be repeated in each vessel. For example, the pulmonary trunk may be ablated twice and/or either or both of the pulmonary arteries may be ablated twice.

The device used during the ablation method may comprises any of the embodiments described herein. Any of the collapsing and deploying methods described herein may be utilized. The movement features described herein may also be utilized in monitoring the location of the distal portion of the catheter 1704 when retracted or otherwise moved within a vessel.

The foregoing description and examples has been set forth merely to illustrate the disclosure and are not intended as being limiting. Each of the disclosed aspects and examples of the present disclosure may be considered individually or in combination with other aspects, examples, and variations of the disclosure. In addition, unless otherwise specified, none of the steps of the methods of the present disclosure are confined to any particular order of performance. Modifications of the disclosed examples incorporating the spirit and substance of the disclosure may occur to persons skilled in the art and such modifications are within the scope of the present disclosure. Furthermore, all references cited herein are incorporated by reference in their entirety.

While the methods and devices described herein may be susceptible to various modifications and alternative forms, specific examples thereof have been shown in the drawings and are herein described in detail. It should be understood, however, that the invention is not to be limited to the particular forms or methods disclosed, but, to the contrary, the invention is to cover all modifications, equivalents, and alternatives falling within the spirit and scope of the various examples described and the appended claims. Further, the disclosure herein of any particular feature, aspect, method, property, characteristic, quality, attribute, element, or the like in connection with an example can be used in all other examples set forth herein. Any methods disclosed herein need not be performed in the order recited. Depending on the example, one or more acts, events, or functions of any of the algorithms, methods, or processes described herein can be performed in a different sequence, can be added, merged, or left out altogether (e.g., not all described acts or events are necessary for the practice of the algorithm). In some examples, acts or events can be performed concurrently, e.g., through multi-threaded processing, interrupt processing, or multiple processors or processor cores or on other parallel architectures, rather than sequentially. Further, no element, feature, block, or step, or group of elements, features, blocks, or steps, are necessary or indispensable to each example. Additionally, all possible combinations, subcombinations, and rearrangements of systems, methods, features, elements, modules, blocks, and so forth are within the scope of this disclosure. The use of sequential, or time-ordered language, such as "then," "next," "after," "subsequently," and the like, unless specifically stated otherwise, or otherwise understood within the context as used, is generally intended to facilitate the flow of the text and is not intended to limit the sequence of operations performed. Thus, some examples may be performed using the sequence of operations described herein, while other examples may be performed following a different sequence of operations.

Conditional language used herein, such as, among others, "can," "might," "may," "e.g.," and the like, unless specifically stated otherwise, or otherwise understood within the context as used, is generally intended to convey that some examples include, while other examples do not include, certain features, elements, and/or states. Thus, such conditional language is not generally intended to imply that features, elements, blocks, and/or states are in any way required for one or more examples or that one or more examples necessarily include logic for deciding, with or without author input or prompting, whether these features, elements and/or states are included or are to be performed in any particular example.

The methods disclosed herein may include certain actions taken by a practitioner; however, the methods can also include any third-party instruction of those actions, either expressly or by implication. For example, actions such as "positioning a catheter" include "instructing positioning of a catheter."

The ranges disclosed herein also encompass any and all overlap, sub-ranges, and combinations thereof. Language such as "up to," "at least," "greater than," "less than," "between," and the like includes the number recited. Numbers preceded by a term such as "about" or "approximately" include the recited numbers and should be interpreted based on the circumstances (e.g., as accurate as reasonably possible under the circumstances, for example ±5%, ±10%, ±15%, etc.). For example, "about 1.5 MHz" includes "1.5 MHz." Phrases preceded by a term such as "substantially" include the recited phrase and should be interpreted based on the circumstances (e.g., as much as reasonably possible under the circumstances). For example, "substantially perpendicular" includes "perpendicular." Unless stated otherwise, all measurements are at standard conditions including temperature and pressure.

The phrase "at least one of' is intended to require at least one item from the subsequent listing, not one type of each item from each item in the subsequent listing. For example, "at least one of A, B, and C" can include A; B; C; A and B; A and C; B and C; or A, B, and C.

## Claims

1. A catheter for ablation of tissue around a vessel, the catheter comprising:
an elongate shaft comprising a distal portion comprising:
a transducer, and
an anchor, wherein the anchor is changeable between a collapsed state and a deployed state,
wherein the anchor in the deployed state is configured to center the transducer in a vessel, to maintain a longitudinal position of the transducer in the vessel, and to preserve blood flow through the vessel.

2. The catheter of claim 1, wherein the anchor is self-expanding.

3. The catheter of claim 1 or 2, wherein the anchor is deployable and collapsible via a push-pull mechanism.

4. The catheter of any one of claims 1 to 3, wherein the anchor comprises a plurality of struts surrounding the transducer.

5. The catheter of any one of claims 1 to 4, wherein the transducer comprises a plurality of arcuate sections.

6. The catheter of any one of claims 1 to 5, wherein the elongate shaft comprises:
an intermediate portion between the proximal portion and the distal portion; and
a suspension between the intermediate portion and the distal portion, the suspension being more flexible than the intermediate portion and the distal portion, the suspension configured to reduce shaft stiffness.

7. The catheter of claim 6, wherein the suspension comprises at least one of a coil or a flexible material.

8. The catheter of any one of claims 1 to 7, wherein the distal portion has a diameter between 3 mm and 10 mm

9. The catheter of any one of claims 1 to 8, wherein the distal portion comprises a Fresnel lens disposed on the transducer,

10. A denervation system comprising:
the catheter of any one of claims 1 to 9; and
an ultrasound system comprising a generator configured to generate an output signal having a frequency between 1.5 MHz and 11 MHz.

11. The denervation system of claim 10, wherein the ultrasound system is configured to adjust the output signal based on data regarding efficiency of the distal portion.

12. A catheter for ablation of tissue around a vessel, the catheter comprising:
an elongate shaft having a diameter between 4.5 mm and 8.5 mm;
a distal portion of the elongate shaft having a delivery state and a deployment state, the distal portion comprising:
an arcuate ultrasound transducer, and
a self-expanding anchor, the anchor in the deployed state configured to center the transducer in a vessel and to maintain a longitudinal position of the transducer in the vessel, and
marker bands; and
an outer sheath surrounding the anchor in the delivery state; and
a handle coupled to a proximal portion of the elongate shaft, the handle comprising an actuator configured to retract the outer sheath, wherein retracting the outer sheath allows the anchor to self-expand from the delivery state to the deployed state confirmable by a change in position of the marker bands.

13. The catheter of claim 12, wherein the anchor is deployable and collapsible via a push-pull mechanism.

14. The catheter of claim 12 or 13, wherein the anchor is configured to preserve blood flow through the vessel.

15. The catheter of any one of claims 11 to 14, wherein the elongate shaft comprises:
an intermediate portion between the proximal portion and the distal portion; and
a suspension between the intermediate portion and the distal portion, the suspension being more flexible than the intermediate portion and the distal portion, the suspension configured to reduce shaft stiffness.
